# EUROPEAN PATENT SPECIFICATION

(11) **EP 0 501 378 B1**
(45) Date of publication and mention of the grant of the patent: **11.01.1995**
(21) Application number: 92103066.4
(22) Date of filing: 24.02.1992
(51) Int. Cl.: C07D 209/20, C07C 229/42, A61K 31/405, A61K 31/19

(54) **NMDA Antagonists**
NMDA-Antagonisten
Antagonistes du NMDA

(30) Priority: 28.02.1991 US 662670
(43) Date of publication of application: 02.09.1992
(60) Divisional application: 94110261.8
(73) Proprietor: MERRELL DOW PHARMACEUTICALS INC., Cincinnati Ohio 45215 (US)
(72) Inventor: Palfreyman, Michael G., Cincinnati, Ohio 45249 (US); McDonald, Ian A., Loveland, Ohio 45140 (US); Salituro, Francesco G., Fairfield, Ohio 45014 (US); Schwarcz, Robert, Baltimore, Maryland 21209 (US)
(74) Representative: VOSSIUS & PARTNER

(56) References cited:
- EP-A- 0 421 946
- WO-A-86/03489
- WO-A-88/09789
- NEUROLOGY vol. 40, no. 4, April 1990, pages 691 - 695; A.FREESE ET AL:'Kynurenine metabolites of tryptophan: implications for neurologic diseases'
- CHEMICAL ABSTRACTS, vol. 112, no. 15, 9 April 1990, Columbus, Ohio, US;abstract no. 135860G, R. LASKE ET AL: 'Investigations on the antiproliferativeeffects of amino acid antagonists targeting for aminoacyl-tRNA synthetases,Part I' page 413 ;

## Description

The present invention is directed to a new class of excitatory amino acid antagonists, their use in the treatment of disease states such as epilepsy, anxiety, stroke, and to pharmaceutical or diagnostic compositions containing these excitatory amino acid antagonists. A further aspect of this invention is directed to the discovery of a new use for a group of known 6-halo-tryptophan and 4-halo-kynurenine derivatives.

WO-880988 discloses that kynurenine is a powerful agonist at the NMDA receptor. WO 8603489 describes N-acylated derivatives of L-tryphtophan with analgenic properties. In EP-A 421946, a document pursuant to Art 54C3) EPC, 6 chloro-tryptophan is described to be an NMDA antagonist. In CA 135860g (1990) tryptophan derivatives are described which inhibit aminoacyl-tRNA synthetase.

In accordance with the present invention, it has been discovered that the following class of tryptophan derivatives are excitatory amino acid antagonists: 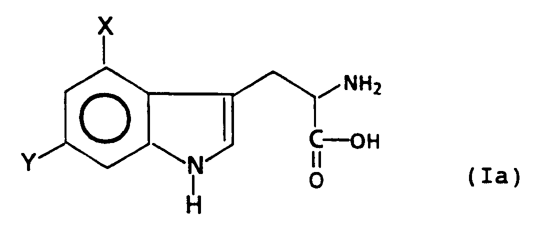
in which X and Y are each independently selected from the group consisting of Cl, Br, F, CH₃, and CH₂CH₃; or a pharmaceutically acceptable salt thereof.

It has also been discovered that the following kynurenine derivatives are excitatory amino acid antagonists: 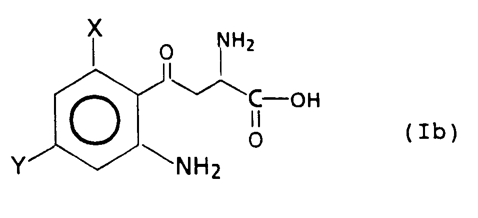
in which X and Y are each independently selected from the group consisting of Cl, Br, F, CH₃, and CH₂CH₃; or a pharmaceutically acceptable salt thereof.

As used in this application:
a) the term "halogen" refers to a fluorine, chlorine, or bromine atom;
b) the term "pharmaceutically acceptable addition salts" refers to either an acid addition or a basic addition salt.

The compounds of Formula Ia, and Ib can exist as either pharmaceutically acceptable acid addition salts or as pharmaceutically acceptable basic addition salts. These compounds may also exist as zwitterions.

The expression "pharmaceutically acceptable acid addition salts" is intended to apply to any non-toxic organic or inorganic acid addition salt of the base compounds represented by Formula Ia or Ib, or any of its intermediates. Illustrative inorganic acids which form suitable salts include hydrochloric, hydrobromic, sulphuric, and phosphoric acid and acid metal salts much as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxy-benzoic, phenylacetic, cinnamic, salicyclic, 2-phenoxy-benzoic, p-toluenesulfonic acid, and sulfonic acids such as methane sulfonic acid and 2-hydroxyethane sulfonic acid. Such salts can exist in either a hydrated or substantially anhydrous form. In general, the acid addition salts of these compounds are soluble in water and various hydrophilic organic solvents, and which in comparison to their free base forms, generally demonstrate higher melting points.

The expression "pharmaceutically acceptable basic addition salts" is intended to apply to any non-toxic organic or inorganic basic addition salts of the compounds represented by Formula Ia, Ib or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine, dimethylamine, trimethylamine, and picoline. Either the mono- or di-basic salts can be formed with those compounds.

All of the compounds of Formula Ia, and Ib contain a chiral center and thus can exist as optical isomers. Any reference to these compounds or their intermediates should be construed as referring to either a racemic mixture or an individual optical isomer. The specific optical isomers can be separated and recovered by techniques known in the art such as chromatography on chiral stationary phases or resolution via chiral salt formation and subsequent separation by selective crystallization. Alternatively utilization of a specific optical isomer as the starting material will produce the corresponding isomer as the final product.

The compounds of Formula Ia are substituted at the 4 and 6 positions as is indicated by the X and Y substituents. X and Y can be represented by the same substituent or by differing substituents. The compounds of Formula Ib are substituted at positions 4 and 6 as is indicated by the X and Y substituents. X and Y can be represented by the same substituents or differing substituents.

Examples of compounds encompassed by Formula Ia include:
4-Bromo-6-fluorotryptophan;
4-Bromo-6-chlorotryptophan;
4-Ethyl-6-bromotryptophan;
4,6-Dibromotryptophan;
4,6-Dichlorotryptophan;
Examples of compounds encompassed by Formula Ib include:
4,6-Dichlorokynurenine;
4-fluoro-6-Bromo-kynurenine;
4-chloro-6-bromokynurenine;
4,6-Dibromo-kynurenine;
6-Ethyl-4-bromokynurenine;
It is preferred for X and Y to each be represented by a halogen atom in either the tryptophans of Ia or the Kynurenines of Ib.

The compounds of Formula Ia may be prepared using techniques and procedures well known and appreciated by one of ordinary skill in the art. A general synthetic procedure for preparing these compounds is set forth in Scheme A. In Scheme A, all substituents unless otherwise indicated are as previously defined. 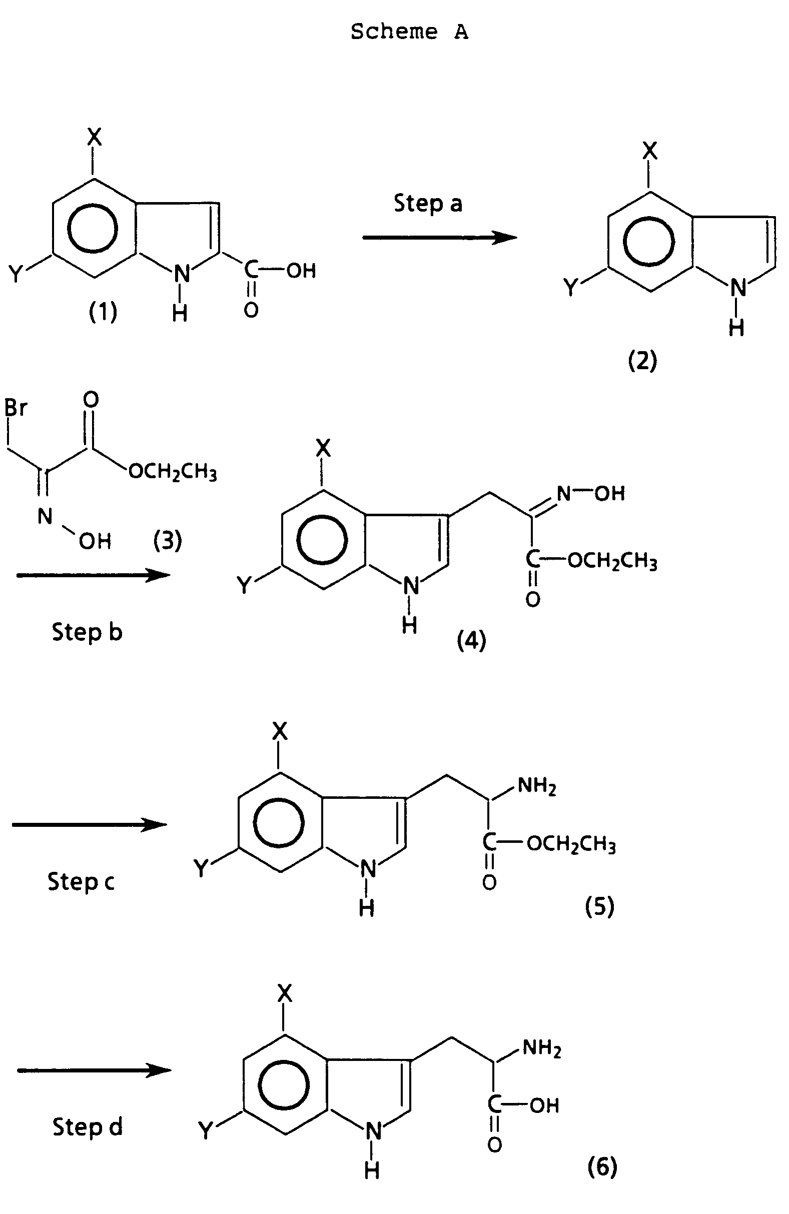
Scheme A provides a general synthetic scheme for preparing compounds of Formula Ia.

In step a, the appropriate 4,6-substituted indole-2-carboxylic acid of structure (1) is decarboxylated to give the corresponding 4,6-substituted indole of structure (2).

For example, the appropriate 4,6-substituted indole-2-carboxylic acid of structure (1) is contacted with a catalytic amount of copper. The reactants are typically contacted in a suitable organic solvent such as quinoline. The reactants are typically stirred together for a period of time ranging from 1-5 hours and at a temperature range of from 200-220°C. The 4,6-substituted indole of structure (2) is recovered from the reaction zone by extractive methods as is known in the art. It may be purified by silica gel chromatography.

In step b, the appropriate 4,6-substituted indole of structure (2) is alkylated with ethyl 3-bromo-2-hydroxyiminopropanoate (3) to give the ethyl 2-(hydroxyimino)-3-(4,6-substituted-3-indolyl)propanoate of structure (4).

For example, the appropriate 4,6-substituted indole of structure (2) is contacted with a molar equivalent of ethyl 3-bromo-2-hydroxyiminopropanoate (3) and a molar excess of a suitable base such as potassium carbonate. The reactants are typically contacted in a suitable organic solvent such as methylene chloride. The reactants are typically stirred at room temperature together for a period of time ranging from 10-50 hours. The ethyl 2-(hydroxyimino)-3-(4,6-substituted-3-indolyl)propanoate of structure (4) is recovered from the reaction zone by extractive methods as is known in the art. It may be purified by silica gel chromatography.

In step c, hydroxyimino functionality of the appropriate ethyl 2-(hydroxyimino)-3-(4,6-substituted-3-indolyl)propanoate of structure (4) is reduced to give the 4,6-substituted tryptophan ethyl ester of structure (5).

For example, the appropriate ethyl 2-(hydroxyimino)-3-(4,6-substituted-3-indolyl)propanoate of structure (4) is contacted with a molar excess of zinc. The reactants are typically contacted in a suitable acidic solvent, such as acetic acid. The reactants are typically stirred together at room temperature for a period of time ranging from 10-100 hours. The 4,6-substituted tryptophan ethyl ester of structure (5) is recovered from the reaction zone by extractive methods as is known in the art. It may be purified by silica gel chromatography.

In step d, the ethyl ester functionality of the appropriate 4,6-substituted tryptophan ethyl ester of structure (5) is removed to give the corresponding 4,6-substituted tryptophan of structure (6).

In order to facilitate the purification of the tryptophan of structure (6), the amino functionality of the appropriate 4,6-substituted tryptophan ethyl ester of structure (5) is first protected as its carbobenzyloxy derivative.

For example, the appropriate 4,6-substituted tryptophan ethyl ester of structure (5) is contacted with molar equivalent of benzyl chloroformate and a slight molar excess of triethylamine. The reactants are typically contacted in a suitable organic solvent such as methylene chloride. The reactants are typically stirred together at room temperature for a period of time ranging from 5-24 hours. The intermediate 4,6-substituted tryptophan-carbobenzyloxy ethyl ester is recovered from the reaction zone by extractive methods as is known in the art. It may be purified by silica gel chromatography.

The ethyl ester functionality of the appropriate intermediate 4,6-substituted tryptophan-carbobenzyloxy ethyl ester is then removed to give the intermediate 4,6-substituted tryptophan-carbobenzyloxy.

For example, the appropriate intermediate 4,6-substituted tryptophan-carbobenzyloxy ethyl ester is contacted with a molar excess of a suitable base such as lithium hydroxide. The reactants are typically contacted in a suitable solvent mixture such as tetrahydrofuran/water. The reactants are typically stirred together at room temperature for a period of time ranging from 1-5 hours. The intermediate 4,6-substituted tryptophan-carbobenzyloxy is recovered from the reaction zone by extractive methods as is known in the art. It may be purified by silica gel chromatography.

The carbobenzyloxy functionality of the appropriate intermediate 4,6-substituted tryptophan-carbobenzyloxycarbonyl is then removed to give the 4,6-substituted tryptophan of structure (6).

For example, the appropriate intermediate 4,6-substituted tryptophan-carbobenzyloxy is contacted with a molar excess of trimethylsilyl iodide. The reactants are typically contacted in a suitable organic solvent such as chloroform. The reactants are typically stirred together at room temperature for a period of time ranging from 1-5 hours. The 4,6-substituted tryptophan of structure (6)is recovered from the reaction zone by extractive methods as is known in the art.

Starting materials for use in Scheme A are readily available to one of ordinary skill in the art. For example, certain 4,6-substituted indole-2-carboxylic acids are described in *J.Med.Chem.* 33 2944-46 **1990**.

The following examples present typical syntheses as described in Scheme A. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "°C" refers to degrees Celsius; "mm Hg" refers to millimeters of mercury; "µL" refers to microliters; "µg" refers to micrograms; and "µM" refers to micromolar.

### Example 1

### DL-4,6-Dichlorotryptophan

### Step a: 4,6-Dichloroindole

Dissolve 4,6-dichloro-indole-2-carboxylic acid (1.0g, 4.35mmol) in quinoline (25mL). Add copper powder (100mg) and heat to 220°C for 3 hours. Pour the resulting black solution into cold concentrated hydrochloric acid (300mL) and extract into ethyl ether (500mL). Filter, wash with 1M hydrochloric acid (2X200mL), water (100mL) and dry (MgSO₄). Evaporate the solvent *in vacuo* to give a brown oil (0.76g). Purify by silica gel chromatography (17% ethyl acetate/hexane) to give the title compound as an amber oil (0.66g, 81%).
¹H NMR (CDCl₃/TMS): 6.56-6.59 ppm (1H, m), 7.10-7.13 ppm (2H, m), 7.16-7.18 ppm (1H, m). ¹³C NMR (CDCl₃), ppm: 101.48, 110.16, 120.24, 125.77, 125.94, 126.54, 122.79, 106.54.

### Step b: Ethyl 2-(hydroxyimino)-3-(4,6-dichloro-3-indolyl)propanoate

Mix 4,6-dichloroindole (5.90g, 31.72mmol), potassium carbonate (1.81g, 47.6mmol) and anhydrous methylene chloride (200mL). Stir and add a solution of ethyl 3-bromo-2-hydroxyiminopropanoate (7.00g, 33.31mmol) in methylene chloride (75mL). Stir under a nitrogen atmosphere for 48 hours. Take the solution up in methylene chloride (100mL) and wash with water (300mL), saturated sodium hydrogen carbonate (200mL) and brine (100mL). Dry (MgSO₄) and evaporate the solvent *in vacuo* to give a tan solid. Purify by silica gel chromatography (1 to 3% acetone in chloroform) to give the title compound as a yellow solid (7.00g, 99% based on consumed starting material). Recrystallize (diethyl ether/hexane) to give the title compound as white crystals (4.0g, 56%); mp 175-176°C
¹H NMR (DMSO-d₆/TMS): 1.19 ppm (3H, t), 4.15 ppm (2H, q), 4.15 ppm (2H, s), 6.95 ppm (1H, s), 7.10 ppm (1H, d), 7.40 ppm (1H, d), 11.3 ppm (1H, bs), 12.35 (1H, s).

| | | | |
|---|---|---|---|
| Anal. Calcd for C₁₃H₁₂Cl₂N₂O₃: | C, 49.54; | H, 3.84; | N, 8.89; |
| Found: | C, 49.30; | H, 3.78; | N, 8.62. |

### Step c: DL-4,6-Dichlorotryptophan ethyl ester

Dissolve ethyl 2-(hydroxyimino)-3-(4,6-dichloro-3-indolyl)propanoate (1.10g, 3.65mmol) in acetic acid (200mL) and add activated zinc dust (1.25g, 19.2mmol). Stir at room temperature for 72 hours. Evaporate the acetic acid in vacuo to give a white oil. Take the white oil up in ethyl acetate (200mL) and treat with saturated sodium hydrogen carbonate (500mL). Filter the resulting white precipitate and separate the organic phase. Wash with saturated sodium hydrogen carbonate (100mL) and brine (100mL). Dry (MgSO₄) and evaporate the solvent *in vacuo* to give the title compound as an amber oil (1.02g, 98%).
¹H NMR (CDCl₃/TMS): 1.25 pppm (3H, t), 1.9 ppm (2H, bs), 3.05 ppm (1H, m), 3.55 ppm (1H, m), 3.9 ppm (1H, m), 4.15 ppm (2H, q), 7.05 ppm (1H, s), 7.05 ppm (1H, d), 7.19 ppm (1H, d), 8.70 ppm (1H, bs).

### Step d: DL-4,6-Dichlorotryptophan

Mix DL-4,6-dichlorotryptophan ethyl ester (2.37g, 7.90mmol) and pyridine (100mL). Add benzyl chloroformate (2.89g, 16.98mmol) and stir for 12 hours. Dilute with ethyl acetate, wash with 1M hydrochloric acid (2X200mL) and brine (2X200mL). Dry (MgSO₄) and evaporate the solvent *in vacuo* to give a yellow oil (3.24g). Purify by recrystallization (hexane/ethyl acetate) to give DL-4,6-dichlorotryptophan-benzyloxycarbonyl ethyl ester as white needles (2.37g, 70%); mp 151-153°C.
¹H NMR (CDCl₃/TMS): 1.20 ppm (3H, t), 3.35 ppm (1H, m), 3.60 ppm (1H, dd), 4.15 ppm (2H, q), 4.75 ppm (1H, m), 5.00 ppm (2H, s), 5.35 ppm (1H, d), 7.00 ppm (1H, s), 7.13 ppm (1H, d), 7.3 ppm (6H, m), 8.10 ppm (1H, bs).

| | | | |
|---|---|---|---|
| Anal. Calcd for C₂₁H₂₀Cl₂N₂O₄: | C, 57.94; | H, 4.63; | N, 6.44; |
| Found: | C, 57.58; | N, 4.33; | N,6.35. |

Mix DL-4,6-dichlorotryptophan-benzyloxycarbonyl ethyl ester (2.10g, 4.88mmol) in 1:1 tetrahydrofuran/water (100mL) and add lithium hydroxide monohydrate (615mg, 14.6mmol). Stir at room temperature for 2 hours. Pour into 1M hydrochloric acid (200mL) and extract with ethyl acetate (200mL). Dry (MgSO₄) and evaporate the solvent *in vacuo* to give DL-4,6-dichlorotryptophan-benzyloxycarbonyl as an amber gum (1.90mg, 100%).
¹H NMR (CDCl₃/TMS): 3.35 ppm (1H, m), 3.65 ppm (1H, dd), 4.75 ppm (1H, m), 5.05 ppm (2H, s), 5.45 ppm (1H, s), 7.00 ppm (1H, s), 7.13 ppm (1H, d), 7.3 ppm (6H, m), 8.35 ppm (1H, bs).
Dissolve DL-4,6-dichlorotryptophan-benzyloxycarbonyl (1.99g, 4.88mmol) in chloroform (100mL) and add trimethylsilyl iodide (4.88g, 24.4mmol). Stir at room temperature for 0.5 hours, quench with methanol and evaporate the solvent *in vacuo* to give a violet oil. Take up the residue in isopropanol (200mL) containing a small amount of DL-dithiothreitol. Neutralize the resulting pale yellow solution with propylene oxide (1.45g, 25mmol) to give a white solid (1.15g, 86%).
FTIR (KBr) cm⁻¹: 3418 (NH), 3033 (COOH), 1616 (C=O), 1586-1479 (Aromatic C=C). ¹H NMR (CDCl₃/TFA/TMS): 3.25 ppm (1H, dd), 4.13 ppm (1H, dd), 4.8 ppm (1H, m), 7.15 ppm (1H, d), 7.2 ppm (1H, s), 7.35 ppm (1H, d), 8.5 ppm (1H, bs). ¹³C NMR (CDCl₃/TFA) ppm: 27.2, 55.4, 106.5, 110.9, 121.7, 122.0, 125.5, 126.9, 129.5, 138.2, 170.25. MS (^{m}/_{z}): 273 (M+, 100%), 255, 237, 227.

| | | | |
|---|---|---|---|
| Anal. Calcd for C₁₁H₉Cl₂N₂O₂·¹/₅H₂O: | C, 47.92; | H, 3.44; | N, 10.16; |
| Found: | C, 47.96; | H, 3.84; | N, 9.97. |

### Example 2

### DL-4,6-Dibromotryptophan

### Step a: 4,6-Dibromoindole

Dissolve 5,7-dibromo-indole-2-carboxylic acid (1.39g, 4.35mmol) in quinoline (25mL). Add copper powder (100mg) and heat to 220°C for 3 hours. Pour the resulting black solution into cold concentrated hydrochloric acid (300mL) and extract into ethyl ether (500mL). Filter, wash with 1M hydrochloric acid (2X200mL), water (100mL) and dry (MgSO₄). Evaporate the solvent *in vacuo* to give the title compound.

### Step b: Ethyl 2-(hydroxyimino)-3-(4,6-dibromo-3-indolyl)propanoate

Mix 4,6-dibromoindole (2.09g, 7.58mmol), potassium carbonate (1.57g, 11.4mmol) and anhydrous methylene chloride (30mL). Stir and add a solution of ethyl 3-bromo-2-hydroxyiminopropanoate (1.59g, 7.58mmol) in methylene chloride (20mL). Stir under a nitrogen atmosphere for 48 hours. Take the solution up in ethyl acetate and wash with water (100mL), saturated sodium hydrogen carbonate (100mL) and brine (100mL). Dry (MgSO₄) and evaporate the solvent *in vacuo* and purify by silica gel chromatography to give the title compound.

### Step c: DL-4,6-Dibromotryptophan ethyl ester

Dissolve ethyl 2-(hydroxyimino)-3-(4,6-dibromo-3-indolyl)propanoate (1.479, 3.65mmol) in acetic acid (200mL) and add activated zinc dust (1.25g, 19.2mmol). Stir at room temperature for 72 hours. Evaporate the acetic acid *in vacuo* and take up in ethyl acetate (200mL). Treat with saturated sodium hydrogen carbonate (500mL). Filter the resulting white precipitate and separate the organic phase. Wash with saturated sodium hydrogen carbonate (100mL) and brine (100mL). Dry (MgSO₄) and evaporate the solvent *in vacuo* to give the title compound.

### Step d: DL-4,6-Dibromotryptophan

Mix DL-4,6-dibromotryptophan ethyl ester (1.20g, 3.07mmol), triethylamine (341mg, 3.38mmol) and methylene chloride (50mL). Add benzyl chloroformate (692mg, 4.06mmol) and stir for 12 hours. Dilute with methylene chloride, wash with 1M hydrochloric acid (100mL) and brine (100mL). Dry (MgSO₄) and evaporate the solvent *in vacuo* and purify by silica gel chromatography to give DL-4,6-dibromotryptophan-benzyloxycarbonyl ethyl ester.
Mix DL-4,6-dibromotryptophan-benzyloxycarbonyl ethyl ester (440mg, 0.84mmol) in 1:1 tetrahydrofuran/water (50mL) and add lithium hydroxide monohydrate (106mg, 2.52mmol). Stir at room temperature for 2 hours. Pour into 1M hydrochloric acid (150mL) and extract with ethyl acetate (200mL). Dry (MgSO₄) and evaporate the solvent *in vacuo* to give DL-4,6-dibromotryptophan-benzyloxycarbonyl.
Dissolve DL-4,6-dibromotryptophan-benzyloxycarbonyl (407mg, 0.82mmol) in chloroform (50mL) and add trimethylsilyl iodide (656mg, 3.28mmol). Stir at room temperature for 1.5 hours and pour the resulting violet solution into isopropanol (50mL) containing a small amount of DL-dithiothreitol. Neutralize with propylene oxide to give a white semi-solid. Take this mixture up in 1M hydrochloric acid (200mL), treat with charcoal and wash with methylene chloride. Evaporate the water *in vacuo*. Dissolve the solid in methanol (50mL) and neutralize with propylene oxide. Add ethyl ether and filter to give the title compound.

The following compounds can be prepared analogously to that described in Example 1 - 2:
DL-4-Bromo-6-fluorotryptophan;
DL-4-Bromo-6-chlorotryptophan;
DL-4-Ethyl-6-bromotryptophan.

The compounds of Formula Ib may be prepared using techniques and procedures well known and appreciated by one of ordinary skill in the art. A general synthetic procedure for preparing these compounds is set forth in Scheme B. In Scheme B, all substituents unless otherwise indicated are as previously defined.

Scheme B provides a general synthetic scheme for preparing compounds of Formula Ib.

In step a, the appropriate 3,5-disubstituted aniline of structure (7) is iodinated to give the corresponding 2-iodo-3,5-disubstituted-aniline of structure (8).

For example, the appropriate 3,5-disubstituted aniline of structure (7) is contacted with a molar equivalent of an appropriate iodinating agent such as N-iodosuccinimide. The reactants are typically contacted in a suitable acidic organic solvent such as acetic acid/methylene chloride. The reactants are typically stirred together at room temperature in the absence of light for a period of time ranging from 5-24 hours. The 2-iodo-3,5-disubstituted-aniline of structure (8) is recovered from the reaction zone by extractive methods as is known in the art. It may be purified by silica gel chromatography.

Alternatively, an appropriate 2-iodo-3,5-disubstituted-aniline of structure (8) may be prepared from an appropriate 2,4-disubstituted-p-nitroaniline. 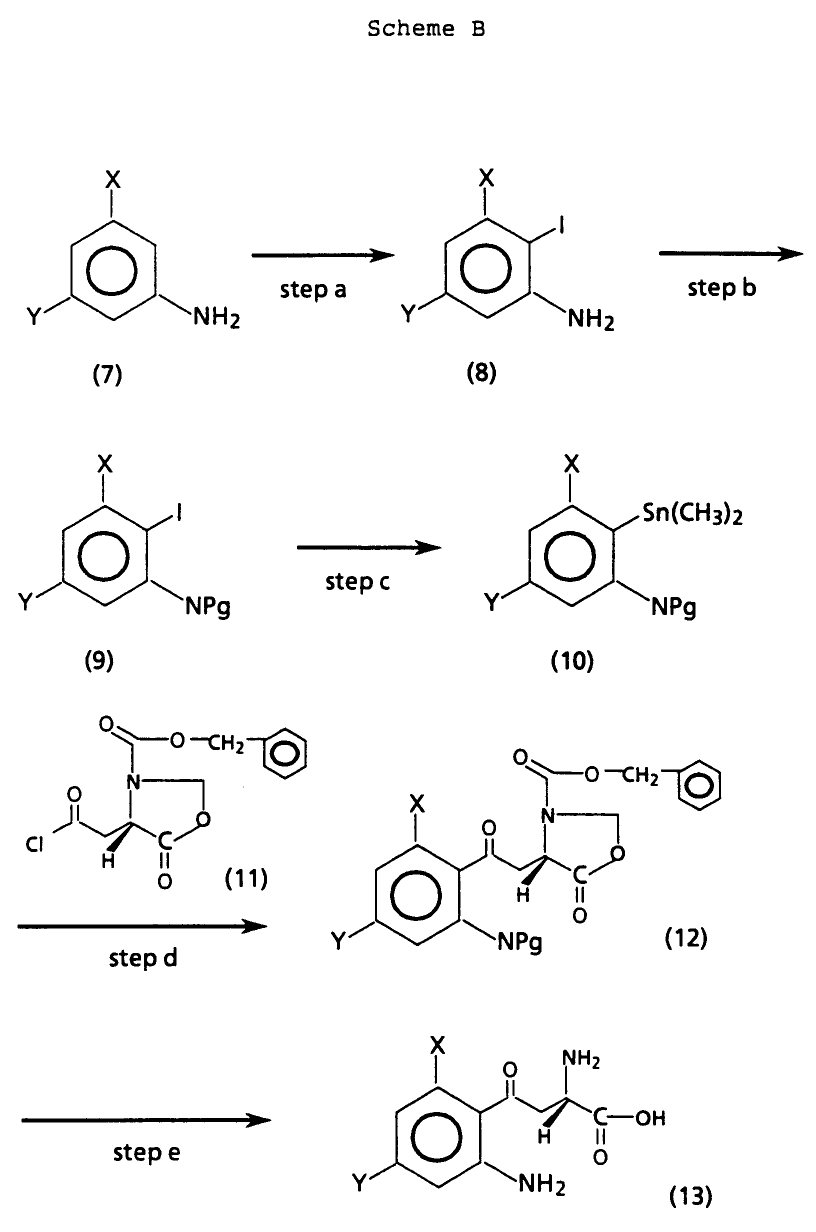
First, an appropriate 2,4-disubstituted-6-nitroaniline is iodinated as is known in the art, such as 50% sulfuric acid, sodium nitrite and potassium iodide, to give the corresponding 2-iodo-3,5-disubstituted-nitrobenzene.

Second, the nitro functionality of an appropriate 2-iodo-3,5-disubstituted-nitrobenzene is reduced as is known in the art, such as tin (II) chloride dihydrate, to give the corresponding 2-iodo-3,5-disubstituted-aniline of structure (8).

In step b, appropriate 2-iodo-3,5-disubstituted-aniline of structure (8) is protected to give the N-protected-3,5-disubstituted-2-iodoaniline of structure (9). The selection and utilization of appropriate protecting groups are well known to one of ordinary skill in the art and are described in "Protective Groups in Organic Synthesis", Theodora W. Greene, Wiley (1981).

In step c, the appropriate N-protected-3,5-disubstituted-2-iodoaniline of structure (9) is converted to the corresponding N-protected-3,5-disubstituted-2-(trimethylstannyl)aniline of structure (10).

For example, the appropriate N-protected-3,5-disubstituted-2-iodoaniline of structure (9) is contacted with a molar excess of an appropriate stannylating agent, such as hexamethylditin, a molar excess of a non-nucleophilic base, such as N-methylmorpholine or sodium hydride, and a catalytic amount of a palladium(0) reagent such as tris(dibenzylideneacetone)dipalladium(0) or Pd(CN)₂Cl₂. The reactants are typically contaced in a suitable organic solvent such as toluene. The reactants are typically stirred together for a period of time ranging from 10-45 hours and at a temperature range of from 40-80°C. The N-protected-3,5-disubstituted-2-(trimethylstannyl)aniline of structure (10) is recovered from the reaction zone and purified by silica gel chromatography.

In step d, the appropriate N-protected-3,5-disubstituted-2-(trimethylstannyl)aniline of structure (10) is reacted with (S)-3-(benzyloxycarbonyl)-5-oxo-4-oxazolidineacetyl chloride (11) to give the corresponding N-protected-2-[1-oxo-2-[(S)-3-(benzyloxycarbonyl)-5-oxo-4-oxazoldine-2-yl]ethyl]-3,5-disubstituted-aniline of structure (12).

For example, the appropriate N-protected-3,5-disubstituted-2-(trimethylstannyl)aniline of structure (10) is contacted with a molar equivalent of (S)-3-(benzyloxycarbonyl)-5-oxo-4-oxazolidineacetyl chloride (11). The reactants are typically contacted in a suitable organic solvent such as toluene. The reactants are typically stirred together for a period of time ranging from 1-5 hours and at a temperature range of from room temperature to 60°C. The N-protected-2-[1-oxo-2-[(S)-3-(benzyloxycarbonyl)-5-oxo-4-oxazolidine-2-yl]ethyl]-3,5-disubstituted-aniline of structure (12) is recovered from the reaction zone and purified by silica gel chromatography.

In step e, the appropriate N-protected-2-[1-oxo-2-[(S)-3-(benzyloxycarbonyl)-5-oxo-4-oxazolidine-2-yl]ethyl]-3,5-disubstituted-aniline of structure (12) is deprotected to give the 4,6-disubstituted-kynurenine of structure (13).

For example, the appropriate N-protected-2-[1-oxo-2-[(S)-3-(benzyloxycarbonyl)-5-oxo-4-oxazolidine-2-yl]ethyl]-3,5-disubstituted-aniline of structure (12) is contacted with a molar excess of trimethylsilyl iodide. The reactants are typically contacted in a suitable organic solvent such as chloroform. The reactants are typically stirred together at room temperature for a period of time ranging from 1-5 hours. The 4,6-disubstituted-kynurenine of structure (13) is recovered from the reaction zone by extractive methods as is known in the art.

Alternatively, the compounds may be sequentially deprotected with TFA to remove the Boc group, in NaOH to deesterify and TMSI to remove the CBZ group.

Starting materials for use in Scheme B are readily available to one of ordinary skill in the art. For example, (S)-3-(benzyloxycarbonyl)-5-oxo-4-oxazolidineacetyl chloride (11) is described in *J.Org.Chem*. 53 6138-39 1988.

The following examples present typical syntheses as described in Scheme B. These examples are understood to be illustrative only and are not intended to limit the scope of the present invention in any way.

### Example 3

### L-4,6-Dichlorokynurenine

### Step a: 2-Iodo-3,5-dichloroaniline

Dissolve 3,5-dichloroaniline (10g, 61.5mmol) in 1:1 acetic acid/methylene chloride (200mL). Add N-iodosuccinimide (16.9g, 61.5mmol) as a solid and stir at room temperature in the absence of light for 8 hours. Pour the solution into saturated sodium hydrogen carbonate (200mL) and extract into methylene chloride (100mL). Wash with saturated sodium hydrogen carbonate (200mL), dry (MgSO₄) and evaporate the solvent *in vacuo*. Purify by silica gel chromatography to give the title compound (8.1g, 45%); mp 61-63°C.
¹H NMR (CDCl₃) 4.4 ppm (2H, bs), 6.70 ppm (1H, d), 6.88 ppm (1H, d); ¹³C NMR (CDCl₃) ppm: 85.77, 111.75, 118.47, 135.21, 139.78, 149.48.

| | | | |
|---|---|---|---|
| Anal. Calcd for C₆H₄Cl₂IN: | C, 25.03; | H, 1.40; | N, 4.87; |
| Found: | C, 25.33; | H, 1.36; | N, 4.87. |

### Step b: N-(t-butoxycarbonyl)-3,5-dichloro-2-iodoaniline

Mix 2-iodo-3,5-dichloroaniline (11.67g, 40.5mmol), di-*tert*-butyldicarbonate (44g, 203mmol) and a catalytic amount of dimethylaminopyridine. Stir for 4 hours at room temperature, take up in ethyl acetate (200mL), wash with 1M hydrochloric acid (200mL), saturated sodium chloride (100mL) and dry (MgSO₄). Evaporate the solvent *in vacuo* and purify by silica gel chromatography to give N-(bis-*t*-butoxycarbonyl)-3,5-dichloro-2-iodoaniline as a white solid (17.4g, 87%); mp 131-132°C.
¹H NMR (CDCl₃/TMS) 1.45 ppm (18H, s), 7.11 ppm (1H, d), 7.41 ppm (1H, d); ¹³C NMR (CDCl₃) ppm: 27.87, 83.22, 103.18, 127.31, 128.07, 134.66, 140.08, 144.80, 149.63.

| | | | |
|---|---|---|---|
| Anal. Calcd for C₁₆H₂₀Cl₂INO₄: | C, 39.37, | H, 4.13; | N, 2.87; |
| Found: | C, 39.37; | H, 4.20; | N, 3.12. |

Mix N-(bis-*t*-butoxycarbonyl)-3,5-dichloro-2-iodoaniline (17.39g, 35.64mmol), potassium carbonate (6.4g, 46mmol) and ethanol (200mL). Heat for 5 hours, pour into water (200mL), extract into ethyl acetate (150mL) and dry (MgSO₄). Evaporate the solvent *in vacuo* and purify by silica gel chromatography (10:1 hexane/ethyl acetate) to give the title compound as a white crystaline solid (12.56g, 91%); mp 64-66°C.
¹H NMR (CDCl₃/TMS) 1.60 ppm (9H, s), 7.1 ppm (1H, bs), 7.19 ppm (1H, d), 8.12 ppm (1H, d).

### Step c: N-(t-butoxycarbonyl)-3,5-dichloro-2-(trimethylstannyl)aniline

Mix sodium hydride (413mg of a 60% suspension in mineral oil, 10.32mmol) and 1-methyl-2-pyrrolidinone (5mL). Place under an argon atmosphere and cool to 0°C. Add a solution of N-(*t*-butoxycarbonyl)-3,5-dichloro-2-iodoaniline (3.33g, 8.60mmol) in 1-methyl-2-pyrrolidinone (50mL). Stir at 0°C for 15 minutes, then for 30 minutes at room temperature. Cool to 0°C and add hexamethylditin (6.7g, 21mmol) followed by Pd(CN)₂Cl₂ (223mg). Stir for 5 minutes, dilute the resulting black solution with diethyl ether (100mL) and pass through a bed of Celite filter aid. Wash the solution with saturated sodium chloride (2X100mL), water (100mL) and dry (MgSO₄). Evaporate the solvent *in vacuo* and purify by silica gel chromatography (14:1 hexane/ethyl acetate) to give the title compound as a white crystalline solid (2.20g, 60%).
¹H NMR (CDCl₃/TMS) 0.50 ppm (9H, s), 1.51 ppm (9H, s), 6.75 ppm (1H, bs), 7.05 ppm (1H, d), 7.70 ppm (1H, d).

### Step d: N-(t-butoxycarbonyl)-2-[1-oxo-2-[(S)-3-(benzyloxycarbonyl)-5-oxo-4-oxazolidine-2-yl]ethyl]-3,5-dichloroaniline

Mix (S)-3-(benzyloxycarbonyl)-5-oxo-4-oxazolidineacetyl chloride (1.85g, 6.21mmol), powdered 4A molecular sieves (4g) and toluene (50mL). Stir under an argon atmosphere for 1 hour. Add a solution of N-(*t*-butoxycarbonyl)-3,5-dichloro-2-(trimethylstannyl)aniline (2.20g, 5.18mmol) in toluene (50mL) followed by Pd(CN)₂Cl₂ (134mg). Heat to 80°C for 1.5 hours, cool and purify directly by silica gel chromatography (3:1 hexane/ethyl acetate) to give the title compound as a colorless oil (1.42g, 53%).
¹H NMR (CDCl₃/TMS) 1.50 ppm (9H, s), 3.50 ppm (1H, dd), 3.8-4.1 ppm (1H, m), 4.5 ppm (1H, bs), 5.25 ppm (2H, dd), 5.35 ppm (1H, dd), 5.5-5.7 ppm (1H, bs), 7.05 ppm (1H, bs), 7.45 ppm (5H, s), 7.85 ppm (1H, d), 8.15 ppm (1H, d).

### Step e: L-4,6-Dichlorokynurenine

Dissolve N-(*t*-butoxycarbonyl)-2-[1-oxo-2-[(S)-3-(benzyloxycarbonyl)-5-oxo-4-oxazolidine-2-yl]ethyl]-3,5-dichloroaniline (700mg, 1.34mmol) in methylene chloride (50mL) and treat with trifluoroacetic acid (20mL). Stir for 3 hours, pour into saturated sodium hydrogen carbonate (100mL) and extract into methylene chloride (100mL). Wash with saturated sodium hydrogen carbonate (100mL), saturated sodium chloride (100mL) and dry (MgSO₄). Evaporate the solvent *in vacuo* and purify by silica gel chromatography (3:1 hexane/ethyl acetate) to give 2-[1-oxo-2-[(S)-3-(benzyloxycarbonyl)-5-oxo-4-oxazolidine-2-yl]ethyl]-3,5-dichloroaniline as a pale yellow oil (380mg, 67%).
¹H NMR (CDCl₃/TMS) 3.60 ppm (1H, dd), 4.0-4.3 ppm (1H, m), 4.45 ppm (1H, bs), 4.9-5.3 ppm (2H, bs), 5.25 ppm (2H, dd), 5.45 ppm (1H, dd), 5.6 ppm (1H, bs), 6.55 ppm (1H, d), 6.70 ppm (1H, d), 7.45 ppm (5H, s).
Dissolve 2-[1-oxo-2-[(S)-3-(benzyloxycarbonyl)-5-oxo-4-oxazolidine-2-yl]ethyl]-3,5-dichloroaniline (380mg, 0.90mmol) in methanol (10mL) and treat with 1N sodium hydroxide (0.99mL, 0.99mmol). Stir for 6 hours, pour into 1M hydrochloric acid (100mL) and extract with ethyl acetate (100mL). Dry (MgSO₄) and evaporate the solvent *in vacuo*. Purify by silica gel chromatography (1 to 5% methanol in chloroform) to give N-(benzyloxycarbonyl)-L-4,6-dichlorokynurenine as a yellow oil (157mg, 43%).
¹H NMR (CDCl₃/TMS) 3.65 ppm (2H, dd), 4.80 ppm (1H, m), 5.15 ppm (2H, dd), 5.9 ppm (1H, d), 6.0-6.2 ppm (2H, bs), 6.50 ppm (1H, d), 6.70 ppm (1H, d), 7.35 ppm (5H, s).
Dissolve N-(benzyloxycarbonyl)-L-4,6-dichlorokynurenine (157mg, 0.43mmol) in chloroform (30mL) and add trimethylsilyl iodide (426mg, 2.13mmol). Stir under an argon atmosphere for 1 hour, quench with methanol and evaporate the solvent *in vacuo*. Take up the resulting red oil in isopropanol (10mL) containing a trace amount of DL-dithiothreitol. Treat the resulting pale yellow solution with propylene oxide (122mg, 2.13mmol) to give the title compound as a yellow solid. Wash with diethyl ether (500mL) and dry at 60°C under 1mm Hg to give the title compound (67mg, 57%).
¹H NMR (DMSO-d6/TMS) 2.90 ppm (1H, dd), 3.20 ppm (1H, dd), 4.85 ppm (1H, dd), 6.6 ppm (1H, d), 6.65 ppm (1H, d), 6.7 ppm (2H, s), 7.5-8.0 ppm (3H, bs); MS (FAB) m/e 277 (M+H, 100), 260 (5), 188 (15).

### Example 4

### 4-fluoro-6-Bromo-kynurenine

### Step a: 2-Iodo-3-bromo-5-fluoroaniline

Mix 4-fluoro-2-nitroaniline (15.6g, 0.1mol) in water (400mL) and add 48% hydrobromic acid (1kg). Add bromine (16g, 0.1mol) with stirring and stir for 1 hour. Dilute to 2L and cool to 7°C. Filter, wash with water and dry to give 4-fluoro-2-bromo-6-nitroaniline.
Mix 4-fluoro-2-bromo-6-nitroaniline (15.5g, 66.0mmol) and 50% sulfuric acid (200mL) and cool to 0-5°C. Add, by dropwise addition, a solution of sodium nitrite (6.0g, 86.4mmol) in water (50mL). Stir for 30 minutes and add solid potassium iodide (29g, 173mmol). Take up the product in ethyl acetate (300mL), wash with saturated sodium hydrogen carbonate (2X200mL), saturated sodium metabisulfite (2X200mL) and water (200mL). Dry (MgSO₄), evaporate the solvent *in vacuo* and purify by silica gel chromatography to give 1-iodo-2-nitro-4-fluoro-6-bromobenzene as a yellow crystalline solid (17.81g; 76%); mp 75-76°C.
¹H NMR (CDCl₃/TMS) 7.35 ppm (1H, dd), 7.65 ppm (1H, dd); ¹³C NMR (CDCl₃) ppm: 111.23, 111.58, 122.98, 123.31, 133.82, 133.94.

| | | | |
|---|---|---|---|
| Anal. Calcd for C₆H₂BrFINO₂: | C, 20.83; | H, 0.58; | N, 4.05; |
| Found: | C, 21.02; | H, 0.74; | N, 4.24. |

Treat 1-iodo-2-nitro-4-fluoro-6-bromobenzene (5.0g, 14.46mmol) with tin (II) chloride dihydrate (12g, 58mmol) in refluxing ethanol (150mL). Stir at reflux for 24 hours, pour into a mixture of ethyl acetate (200mL) and saturated sodium hydrogen carbonate (500mL). Filter and wash the filtrate with saturated sodium hydrogen carbonate (200mL) and water (200mL). Dry (MgSO₄), evaporate the solvent *in vacuo* and purify by silica gel chromatography to give the title compound as an off-white solid (3.45g, 76%).
¹H NMR (CDCl₃/TMS) 4.5 ppm (2H, bs), 6.45 ppm (1H, dd), 7.85 ppm (1H, dd).

### Step b: N-(t-butoxycarbonyl)-3-bromo-5-fluoro-2-iodoaniline

Mix 2-Iodo-3-bromo-5-fluoroaniline (3.00g, 9.5mmol), di-*tert*-butyldicarbonate (4.15g, 19mmol) and a catalytic amount of dimethylaminopyridine. Stir for 4 hours at room temperature, take up in ethyl acetate, wash with 1M hydrochloric acid, saturated sodium chloride and dry (MgSO₄). Evaporate the solvent *in vacuo* and purify by silica gel chromatography to give N-(bis-*t*-butoxycarbonyl)-3-bromo-5-fluoro-2-iodoaniline as a white solid (4.36g, 90%).
¹H NMR (CDCl₃/TMS) 1.45 ppm (18H, s), 6.95 ppm (1H, dd), 7.40 ppm (1H, dd).
Mix N-(bis-*t*-butoxycarbonyl)-3-bromo-5-fluoro-2-iodoaniline (4.00g, 7.75mmol), potassium carbonate (10mmol) and ethanol (50mL). Heat for 5 hours, pour into water (50mL), extract into ethyl acetate (40mL) and dry (MgSO₄). Evaporate the solvent *in vacuo* and purify by silica gel chromatography to give the title compound as a white crystaline solid (2.66g, 83%).
¹H NMR (CDCl₃/TMS) 1.55 ppm (9H, s), 7.15 ppm (1H, dd), 7.20 ppm (1H, bs), 7.95 ppm (1H, dd).

### Step c: N-(t-butoxycarbonyl)-3-bromo-5-fluoro-2-(trimethylstannyl)aniline

Mix sodium hydride (317mg of a 60% suspension in mineral oil, 7.9mmol) and 1-methyl-2-pyrrolidinone (5mL). Place under an argon atmosphere and cool to 0°C. Add a solution of N-(*t*-butoxycarbonyl)-3-bromo-5-fluoro-2-iodoaniline (2.50g, 6.10mmol) in 1-methyl-2-pyrrolidinone (50mL). Stir at 0°C for 15 minutes, then for 30 minutes at room temperature. Cool to 0°C and add hexamethylditin (9.8g, 30mmol) followed by Pd(CN)₂Cl₂ (223mg). Stir for 5 minutes, dilute the resulting black solution with diethyl ether (100mL) and pass through a bed of Celite filter aid. Wash the solution with saturated sodium chloride (2X100mL), water (100mL) and dry (MgSO₄). Evaporate the solvent *in vacuo* and purify by silica gel chromatography to give the title compound as a white solid (1.50g, 54%).
¹H NMR (CDCl₃/TMS) 0.50 ppm (9H, s), 1.50 ppm (9H, s), 6.80 ppm (1H, bs), 7.00 ppm (1H, dd), 7.65 ppm (1H, dd).

### Step d: N-(t-butoxycarbonyl)-2-[1-oxo-2-[(S)-3-(benzyloxycarbonyl)-5-oxo-4-oxazolidine-2-yl]ethyl]-3-bromo-5-fluoroaniline

Mix (S)-3-(benzyloxycarbonyl)-5-oxo-4-oxazolidineacetyl chloride (946g, 3.18mmol), powdered 4A molecular sieves (4g) and toluene (50mL). Stir under an argon atmosphere for 1 hour. Add a solution of N-(*t*-butoxycarbonyl)-3-bromo-5-fluoro-2-(trimethylstannyl)aniline (1.44g, 3.18mmol) in toluene (50mL) followed by Pd(CN)₂Cl₂ (134mg). Heat to 80°C for 1.5 hours, cool and purify directed by silica gel chromatography (3:1 hexane/ethyl acetate) to give the title compound as a white crystalline solid (123mg, 7%).
¹H NMR (CDCl₃/TMS) 1.55 ppm (9H, s), 3.45-4.25 ppm (2H, m), 4.45 ppm (1H, bs), 5.20 ppm (2H, m), 5.4-5.6 ppm (2H, m), 7.00 ppm (1H, m), 7.35-7.45 ppm (6H, bs), 7.90 ppm (1H, m).

### Step e: L-6-Bromo-4-fluorokynurenine

Dissolve N-(*t*-butoxycarbonyl)-2-[1-oxo-2-[(S)-3-(benzyloxycarbonyl)-5-oxo-4-oxazolidine-2-yl]ethyl]-3-bromo-5-fluoroaniline (1.34mmol) in methylene chloride (50mL) and treat with trifluoroacetic acid (20mL). Stir for 3 hours, pour into saturated sodium hydrogen carbonate (100mL) and extract into methylene chloride (100mL). Wash with saturated sodium hydrogen carbonate (100mL), saturated sodium chloride (100mL) and dry (MgSO₄). Evaporate the solvent *in vacuo* and purify by silica gel chromatography (3:1 hexane/ethyl acetate) to give 2-[1-oxo-2-[(S)-3-(benzyloxycarbonyl)-5-oxo-4-oxazolidine-2-yl]ethyl]-3-bromo-5-fluoroaniline.
Dissolve 2-[1-oxo-2-[(S)-3-(benzyloxycarbonyl)-5-oxo-4 oxazolidine-2-yl]ethyl]-3-bromo-5-fluoroaniline (0.90mmol) in methanol (10mL) and treat with 1N sodium hydroxide (0.99mL, 0.99mmol). Stir for 6 hours, pour into 1M hydrochloric acid (100mL) and extract with ethyl acetate (100mL). Dry (MgSO₄) and evaporate the solvent *in vacuo*. Purify by silica gel chromatography (1 to 5% methanol in chloroform) to give N-(benzyloxycarbonyl)-L-6-bromo-4-fluorokynurenine.
Dissolve N-(benzyloxycarbonyl)-L-6-bromo-4-fluorokynurenine (0.43mmol) in chloroform (30mL) and add trimethylsilyl iodide (426mg, 2.13mmol). Stir under an argon atmosphere for 1 hour, quench with methanol and evaporate the solvent *in vacuo*. Take up the resulting residue in isopropanol (10mL) containing a trace amount of DL-dithiothreitol. Treat the solution with propylene oxide (122mg, 2.13mmol) to give the title compound.

The following compounds can be prepared analogously to those described in Examples 3 and 4:
6-Bromo-4-chlorokynurenine;
6-Bromo-4-bromokynurenine;
6-Ethyl-4-bromokynurenine.

An alternate synthetic procedure for preparing compounds of Formula Ib is outlined in Scheme C. In Scheme C, all substituents unless otherwise indicated, are as previously defined.

Scheme C provides an alternate synthetic procedure for preparing compounds of Formula Ib.

In step a, the appropriate 4,6-substituted tryptophan ethyl ester of structure (5) is protected to give the 4,6-substituted-tryptophan-benzyloxycarbonyl ethyl ester of structure (14) as described previously in Scheme A, step d.

In step b, the appropriate 4,6-substituted tryptophan-benzylcarbonyl ethyl ester of structure (14) is oxidatively cleaved to give the N-(benzyloxycarbonyl)-3,5-disubstituted-kynurenine ethyl ester of structure (15).

For example, the appropriate 4,6-substituted tryptophan-benzyloxycarbonyl ethyl ester of structure (14) is contacted with a molar excess of an oxidating agent such as 4-t-butyl iodylbenzene. The reactants are typically contacted in a suitable organic solvent such as chlorobenzene. The reactants are typically stirred together for a period of time ranging from 2-24 hours and at a temperature range of from room temperature to reflux. The N-(benzyloxycarbonyl)-3,5-disubstituted-kynurenine ethyl ester of structure (15) is recovered from the reaction zone by evaporate of the solvent. It can be purified by silica gel chromatography. 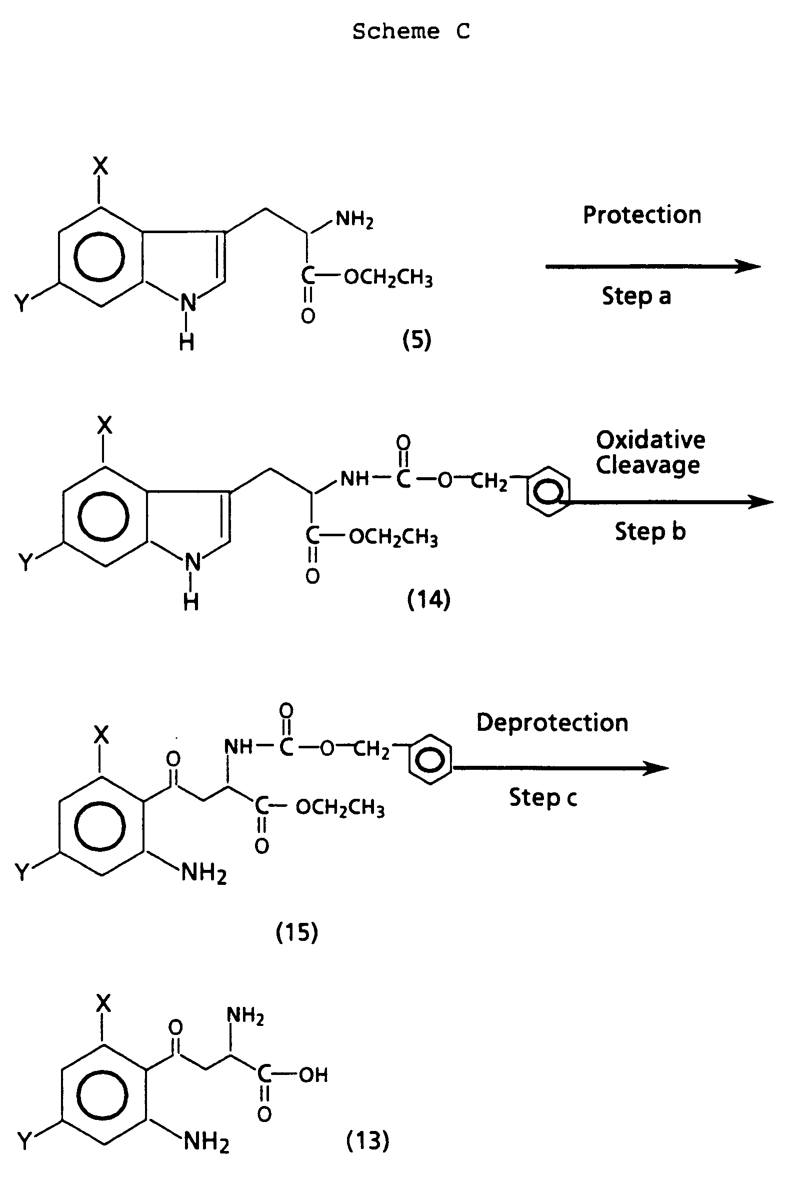
Alternatively, the appropriate 4,6-disubstituted tryptophan-benzylcarbonyl ethyl ester of structure (14) may be oxidatively cleaved with ozone as is known in the art to give an intermediate N-(benzyloxycarbonyl)-3,5-disubstituted-N-formyl-kynurenine. The N-formyl functionality of the intermediate N-(benzyloxycarbonyl)-3,5-disubstituted-N-formyl-kynurenine is then removed by acid hydrolysis to give the corresponding N-(benzyloxycarbonyl)-3,5-disubstituted-kynurenine ethyl ester of structure (15).

In step c, the protecting groups of the appropriate N-(benzyloxycarbonyl)-3,5-disubstituted-kynurenine ethyl ester of structure (15) are removed to give the corresponding 3,5-disubstituted-kynurenine of structure (13) as described previously in Scheme A, step d.

Starting materials for use in Scheme C are readily available to one of ordinary skill in the art. For example, 4-t-butyl iodylbenzene is described in *J.Chem.Soc., Chem. Commun.* 1887-88 .

The following examples present typical syntheses as described in Scheme C. These examples are understood to be illustrative only and are not intended to limit the scope of the present invention in any way.

### Example 5

### 4,6-Dibromokynurenine

### Step a: N-(Benzyloxycarbonyl)-4,6-dibromotryptophan ethyl ester

Mix DL-4,6-dibromotryptophan ethyl ester (1.20g, 3.07mmol), triethylamine (341mg, 3.38mmol) and methylene chloride (50mL). Add benzyl chloroformate (692mg, 4.06mmol) and stir for 12 hours. Dilute with methylene chloride, wash with 1M hydrochloric acid (100mL) and brine (100mL). Dry (MgSO₄) and evaporate the solvent *in vacuo* and purify by silica gel chromatography to give the title compound.

### Step b: N-(Benzyloxycarbonyl)-4,6-dibromokynurenine ethyl ester

Dissolve N-(benzyloxycarbonyl)-4,6-dibromotryptophan ethyl ester (1.05g, 2mmol) in chlorobenzene (8mL) and mix with 4-t-butyl iodylbenzene (876g, 3mmol). Reflux for 4 hours and evaporate the solvent in vacuo. Purify by silica gel chromatography to give the title compound.

### Step c: 4,6-Dibromokynurenine

Mix N-(benzyloxycarbonyl)-4,6-dibromokynurenine ethyl ester (444mg, 0.84mmol) with 1:1 tetrahydrofuran/water (50mL) and add lithium hydroxide monohydrate (106mg, 2.52mmol). Stir at room temperature for 2 hours. Pour into 1M hydrochloric acid (150mL) and extract with ethyl acetate (200mL). Dry (MgSO₄) and evaporate the solvent *in vacuo* to give N-(benzyloxycarbonyl)-4,6-dibromokynurenine.
Dissolve N-(benzyloxycarbonyl)-4,6-dibromokynurenine (410mg, 0.82mmol) in chloroform (50mL) and add trimethylsilyl iodide (656mg, 3.28mmol). Stir at room temperature for 1.5 hours and pour the resulting violet solution into isopropanol (50mL) containing a small amount of DL-dithiothreitol. Neutralize with propylene oxide to give a white semi-solid. Take this mixture up in 1M hydrochloric acid (200mL), treat with charcoal and wash with methylene chloride. Evaporate the water *in vacuo* to give a white solid. Dissolve the white solid in methanol (50mL) and neutralize with propylene oxide. Add ethyl ether and filter to give the title compound.

### Example 6

### 4,6-Dichlorokynurenine (See Example 3)

### Step b: N-(Benzyloxycarbonyl)-4,6-dichlorokynurenine ethyl ester

Dissolve DL-4,6-dichlorotryptophan-benzyloxycarbonyl ethyl ester (1.90g, 4.42mmol) in methanol (200mL), cool to -78°C and treat with ozone until a blue color is observed (approximately 3-5 minutes). Purge with nitrogen gas and quench with dimethylsulfide (10mL). Evaporate the solvent *in vacuo,* take up in diethyl ether and wash with water (2X150mL) and brine (200mL). Dry (MgSO₄) and evaporate the solvent *in vacuo* to give N-(formyl)-2-[ethyl-4-oxo-2-(benzyloxycarbonylamino)butyrate-4-yl]-3,5-dichloroaniline as an amber oil (1.98g, 96%).
¹H NMR (CDCl₃/TMS) 1.25 ppm (3H, t), 3.55 ppm (2H, d), 4.25 (2H, q), 5.80 ppm (1H, m), 5.15 ppm (2H, s), 5.80 ppm (1H, bs), 7.19 ppm (1H, d), 7.35 ppm (5H, s), 8.30 ppm (1H, d), 8.40 ppm (1H, s), 8.75 ppm (1H, bs).
Dissolve N-(formyl)-2-[ethyl-4-oxo-2-(benzyloxycarbonylamino)butyrate-4-yl]-3,5-dichloroaniline (1.94g, 4.15mmol) in methanol (200mL) and treat with 4N hydrochloric acid/dioxane (8.3mmol). Stir for 1 hour, pour into saturated sodium hydrogen carbonate (200mL) and extract into ethyl acetate (150mL). Dry (MgSO₄) and evaporate the solvent *in vacuo* to give the title compound as a yellow oil (1.82g, 100%)
¹H NMR (CDCl₃/TMS) 1.35 ppm (3H, t), 3.65 ppm (2H, dd), 4.25 ppm (2H, q), 4.70 ppm (1H, m), 5.15 ppm (4H, bs), 5.80 ppm (1H, d), 6.55 ppm (1H, d), 6.70 ppm (1H, d), 7.35 ppm (5H, s).

### Step c: 4,6-Dichlorokynurenine

Dissolve N-(benzyloxycarbonyl)-4,6-dichlorokynurenine ethyl ester (1.82g, 4.14mmol) in a 1:1 mixture of tetrahydrofuran/water (100mL). Treat with lithium hydroxide monohydrate (522mg, 12.4mmol) and warm at 70°C for 4 hours. Pour into 1M hydrochloric acid (100mL) and extract into ethyl acetate (150mL). Dry (MgSO₄) and evaporate the solvent *in vacuo* to give N-(benzyloxycarbonyl)-4,6-dichlorokynurenine as a yellow foam (1.52g, 89%).
¹H NMR (CDCl₃/TMS) 3.50-3.80 ppm (2H, dd), 4.80 ppm (1H, m), 5.15 ppm (2H, bs), 6.0 ppm (1H, d), 6.55 ppm (1H, d), 6.70 ppm (1H, d), 7.00 ppm (3H, bs), 7.35 ppm (5H, s).
Dissolve N-(benzyloxycarbonyl)-4,6-dichlorokynurenine (1.52g, 3.70mmol) in chloroform (100mL) and add trimethylsilyl iodide (3.7g, 18.5mmol). Stir at room temperature under an argon atmosphere for 1 hour, quench with methanol and evaporate the solvent *in vacuo.* Take up the resulting red oil into isopropanol (10mL) containing a small amount of DL-dithiothreitol. Neutralize the resulting pale yellow solution with propylene oxide (1.0g, 18.5mmol) to give a yellow solid. Wash with diethyl ether (500mL) and dry at 60°C under 1 mm Hg to give the title compound (870mg, 85%).
¹H NMR (DMSO-d6/TMS) 2.90 ppm (1H, dd), 3.15-3.25 ppm (1H, m), 3.85 ppm (1H, dd), 6.60 ppm (1H, d), 6.70 ppm (1H, d), 6.75 ppm (2H, s), 7.6-7.9 ppm (3H, bs); MS (FAB) m/e 277 (M+H, 100), 260 (15), 188 (55); HRMS (FAB) Calcd. for C₁₀H₁₁Cl₂N₂O₃: M+H 277.01467. Found: M+H 277.0160.

As noted above, the compounds of Formula Ia, and Ib (hereinafter "the compounds") antagonize the effects which excitatory amino acids have upon the NMDA receptor complex. This antagonist effect can be demonstrated by their ability to prevent NMDA-stimulated cyclic GMP accumulation in neonatal rat cerebellar tissue. This test is based upon the phenomenon that when samples of neonatal rat cerebellar tissue are exposed to the agonist, NMDA, there is an increase in cyclic GMP levels within this tissue. NMDA antagonists inhibit or decrease this rise in cyclic GMP levels. This test can be performed by methods similar to those of Baron et al., J. Pharmacol. Exp. Ther. Vol 250 page 162 (1989).

The compounds exhibit anti-convulsant properties and pharmaceutical compositions containing them are useful in the treatment of epilepsy. They are useful in the treatment of grand mal seizures, petit mal seizures, psychomotor seizures, autonomic seizures, etc. One method of demonstrating their anti-epileptic properties is by their ability to inhibit the seizures that are caused by the administration of quinolinic acid, an NMDA agonist. This test can be conducted in the following manner.

One group containing ten mice are administered 0.01 - 100 µg of test compound intracerebroventricularly in a volume of 5 microliters of saline. A second control group containing an equal number of mice are administered an equal volume of saline as a control. Approximately 5 minutes later, both groups are administered 7.7 micrograms of quinolinic acid intracerebroventricularly in a volume of 5 microliters of saline. The animals are observed for 15 minutes thereafter for signs of clonic-tonic seizures. The control group will have a statistically higher rate of clonic-tonic seizures than will the test group.

The compounds are useful for preventing or minimizing the damage which nervous tissues contained within the CNS suffer upon exposure to either ischemic, hypoxic, or hypoglycemic conditions or as the result of physical trauma. Representative examples of such conditions include strokes or cerebrovascular accidents, hyperinsulinemia, cardiac arrest, physical trauma, drownings, suffocation, and neonatal anoxic trauma. The pharmaceutical compositions containing the compounds should be administered to the patient within 24 hours of the onset of the hypoxic, ischemic, or hypoglycemic condition in order for the compounds to effectively minimize the CNS damage which the patient will experience.

The pharmaceutical compositions containing the compounds are also useful in the treatment of neurodegenerative diseases such as Huntington's disease, Alzheimer's disease, senile dementia, glutaric acidaemia type I, Parkinson's disease, multi-infarct dementia, and neuronal damage associated with uncontrolled seizures. The administration of these compounds to a patient experiencing such a condition will serve to either prevent the patient from experiencing further neurodegeneration or it will decrease the rate at which the neurodegeneration occurs.

As is apparent to those skilled in the art, the compounds will not correct any CNS damage that has already occurred as the result of either disease, or a lack of oxygen or sugar. As used in this application, the term "treat" refers to the ability of the compounds to prevent further damage or delay the rate at which any further damage occurs.

The compounds exhibit an anxiolytic effect and pharmaceutical compositions containing them are thus useful in the treatment of anxiety. The compounds also exhibit an analgesic effect and the pharmaceutical compositions containing them are useful in controlling pain. They may be co-administered with a narcotic analgesic such as morphine, demerol, etc. In addition to lowering the dose of narcotic required, a decrease in the rate at which patients develop tolerance to the pharmacological effects of these narcotics is achieved. It is also believed that this co-administration will help to prevent the patient from becoming addicted to the narcotic.

They are also effective in the treatment of migraine. They can be used prophylactically or to relieve the symptoms associated with a migraine episode.

In order to exhibit these therapeutic properties, pharmaceutical compositions containing the compounds need to be administered in a quantity sufficient to inhibit the effect which the excitatory amino acids have upon the NMDA receptor complex. The dosage range at which these compounds exhibit this antagonistic effect can vary widely depending upon the particular disease being treated, the severity of the patient's disease, the patient, the particular compound being administered, the route of administration, and the presence of other underlying disease states within the patient, etc. Typically the compounds exhibit their therapeutic effect at a dosage range of from about 0.1 mg/kg/day to about 100 mg/kg/day for any of the diseases or conditions listed above. Repetitive daily administration may be desirable and will vary according to the conditions outlined above.

It has been discovered that probenecid will potentiate the therapeutic activity of the excitatory amino acid antagonists of the present invention. Thus the compounds will exhibit their therapeutic effects at lower doses and for longer periods in patients who are concurrently receiving probenecid. The mechanism by which probenecid potentiates their effects is not fully understood, however it is believed that probenecid decreases the rate at which the compounds are removed from the central nervous system as well as decreasing the rate of excretion by the kidneys. Probenecid increases the effective concentration of these compounds in both the CNS and in the systemic circulation.

Probenecid is known in the art. It is available commercially from Merck Sharp and Dohme under the tradename Benemid® as well as being available from numerous other sources. Probenecid is a uricosuric agent and is utilized in the treatment of gout. Probenecid is a renal tubular transport blocking agent and has been utilized to increase plasma levels of penicillin. The pharmacology of probenecid is described in detail in the 45th Edition of the Physicians Desk reference on page 1379. Probenecid is currently available commercially as tablets. The sodium salt of probenecid is readily water soluble and injectable dosage form can be prepared from this salt using techniques well known to those skilled in the art.

The pharmaceutical compositions containing the compounds of the invention may be administered concurrently with probenecid in order to treat any of the diseases or conditions described above. The quantity of probenecid that is required to potentiate the therapeutic effects of the compounds can vary widely depending upon the particular compound being administered, the patient, and the presence of other underlying disease states within the patient, etc. Typically though, the probenecid may be administered at a dosage of from 0.5-3g/day. Repetitive daily administration may be desirable and will vary according to the conditions outlined above. The probenecid will typically be administered from 2-4 times daily.

With the concurrent administration of probenecid, the dosage range for the excitatory amino antagonists may be adjusted lower by a factor of from 2-10. Alternatively, the compounds of Formulae Ia or Ib may be administered at the same dosage range in order to obtain an enhanced effect due to the higher therapeutic concentrations obtained.

The pharmaceutical compositions containing the compounds of the present invention may be administered by a variety of routes. They are effective if administered orally. They may also be administered parenterally (i.e. subcutaneously, intravenously, intramuscularly, intraperitoneally, or intrathecally).

Pharmaceutical compositions can be manufactured utilizing techniques known in the art. Typically an antagonistic amount of the compound will be admixed with a pharmaceutically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations.

In another embodiment, the compounds can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds of Formulae Ia, or Ib, and the probenecid can be administered as two different pharmaceutical dosage forms. Alternatively, in order to increase patient convenience, the compounds and the probenecid may be compounded into a single pharmaceutical dosage form. These pharmaceutical compositions can be manufactured utilizing techniques known in the art similar to those described above. Typically an antagonistic amount of the compound of Formula I and an effective amount of probenecid will be admixed with a pharmaceutically acceptable carrier.

As used in this application:
a) the term "patient" refers to warm blooded animals such as, for example, guinea pigs, mice, rats, cats, rabbits, dogs, monkeys, chimpanzees, and humans;
b) the term "treat" refers to the ability of the compounds to either relieve, alleviate, or slow the progression of the patient's disease or prophylactically prevent its occurrence or the manifestation of its symptoms;
c) the phrase "antagonize the effects of excitatory amino acids" and the phrase "excitatory amino acid antagonist" should be referred to the ability of the compounds to inhibit or decrease the rate at which glutamate or glycine produce neurotransmission at the NMDA receptor complex, and;
d) the term "neurodegeneration" refers to a progressive death and disappearance of a population of nerve cells occurring in a manner characteristic of a particular disease state and leading to brain damage;
e) the phrase "concurrent administration" refers to administering the probenecid at an appropriate time so that it will potentiate the antagonistic effects of the compounds of Formula Ia or Ib. This may means simultaneous administration or administration at appropriate but different times. Establishing such a proper dosing schedule will be readily apparent to one skilled in the art.

The compounds may also be admixed with any inert carrier and utilized in laboratory assays in order to determine the concentration of the compounds within the serum, urine, etc., of the patient as is known in the art.

Neurodegenerative diseases are typically associated with a dysfunction of NMDA receptors. Thus, the compounds of Formula I may be utilized in diagnostic procedures to aid physicians with the diagnosis of neurodegenerative diseases.
The compounds may be labeled with imaging agents known in the art such as isotopic atoms and administered to a patient in order to determine whether the patient is exhibiting a decreased number of NMDA receptors and the rate at which that loss is occurring.

## Claims (Claims for the following Contracting State(s): AT, BE, CH, DE, DK, FR, GB, IT, LI, LU, MC, NL, PT, SE)

1. **.** Compounds of the formulae: 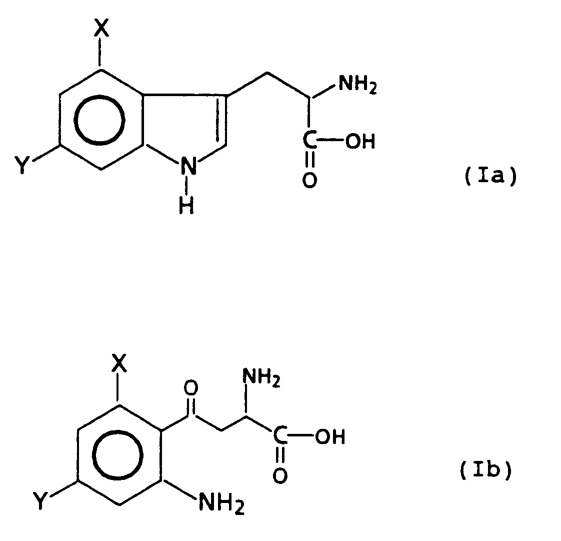 in which X and Y are each independently represented by a substituent selected from the group consisting of Cl, Br, F, CH₃, and CH₂CH₃ or a a pharmaceutically acceptable salt thereof.

2. A compound according to claim 1 in which X and Y are each represented by halogen atoms.

3. A pharmaceutical composition comprising a compound according to claim 1 or 2 in admixture with a pharmaceutically acceptable carrier.

4. A pharmaceutical composition according to claim 3 useful in antagonizing the effects of excitatory amino acids upon the NMDA receptor complex.

5. A pharmaceutical composition according to claim 4 useful in the treatment of epilepsy.

6. A pharmaceutical composition according to claim 4 useful in the treatment of neurodegenerative diseases.

7. A pharmaceutical composition according to claim 4 useful in preventing ischemic/hypoxic/hypoglycemic damage to cerebral tissue.

8. A pharmaceutical composition according to claim 4 useful in the treatment of anxiety.

9. A pharmaceutical composition according to claim 4 useful in producing an analgesic effect.

10. A pharmaceutical composition according to claim 4 useful in the treatment of migraine.

11. A pharmaceutical composition comprising a pharmaceutical composition according to any one of claims 3 to 10 in admixture with probenecid.

12. A pharmaceutical composition according to any one of claims 3 to 10 comprising additionally probenecid in a separate dosage form for concurrent administration.

## Claims (Claims for the following Contracting State(s): ES)

1. A process for the preparation of compounds of the formulae: 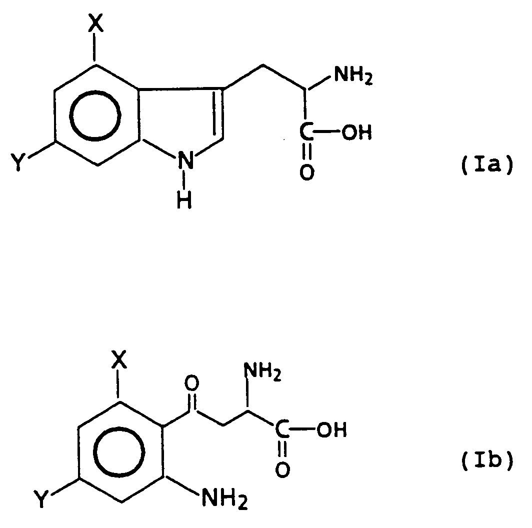 in which X and Y are each independently represented by a substituent selected from the group consisting of Cl, Br, F, CH₃, and CH₂CH₃ or a a pharmaceutically acceptable salt thereof,
comprising:
a) for those compounds Ia by carrying out the reaction depicted in Reaction Scheme A in which X and Y are as above: 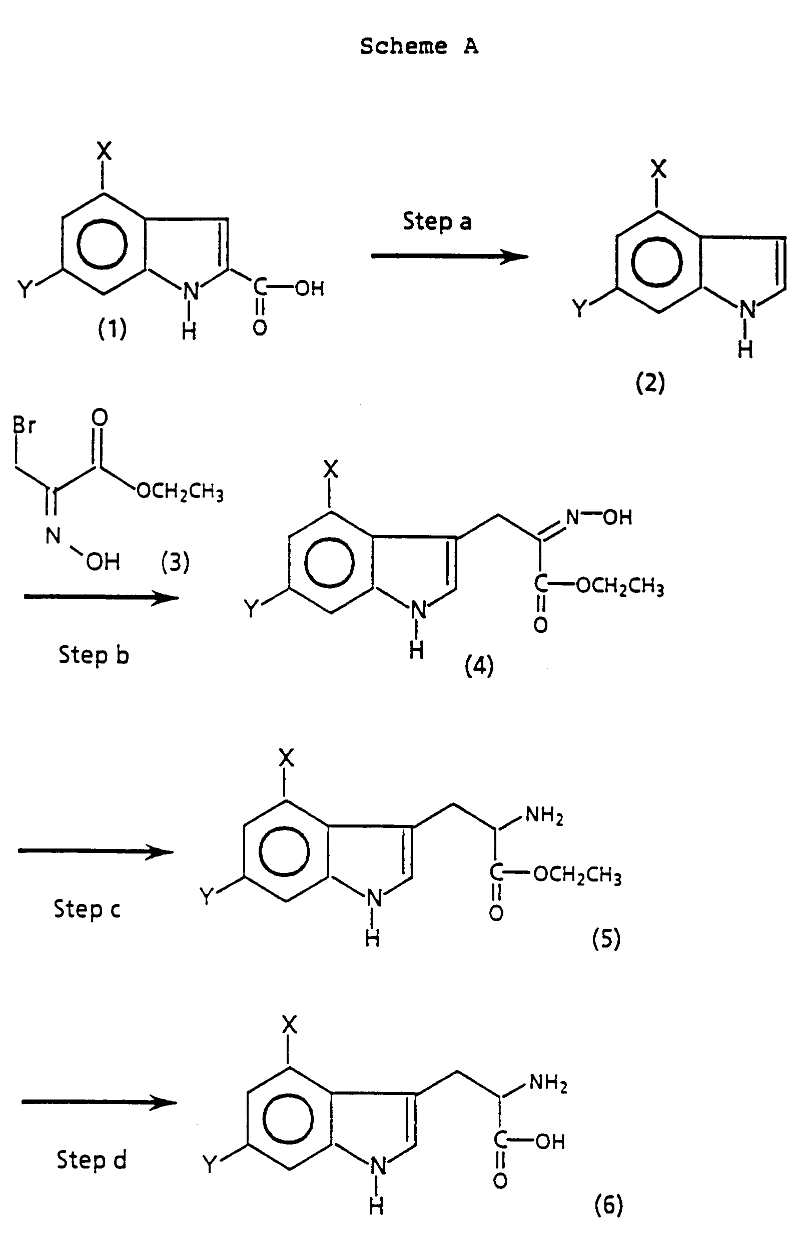
b) for those compounds Ib by carrying out the reaction depicted in Reaction Scheme B in which X and Y are as above: 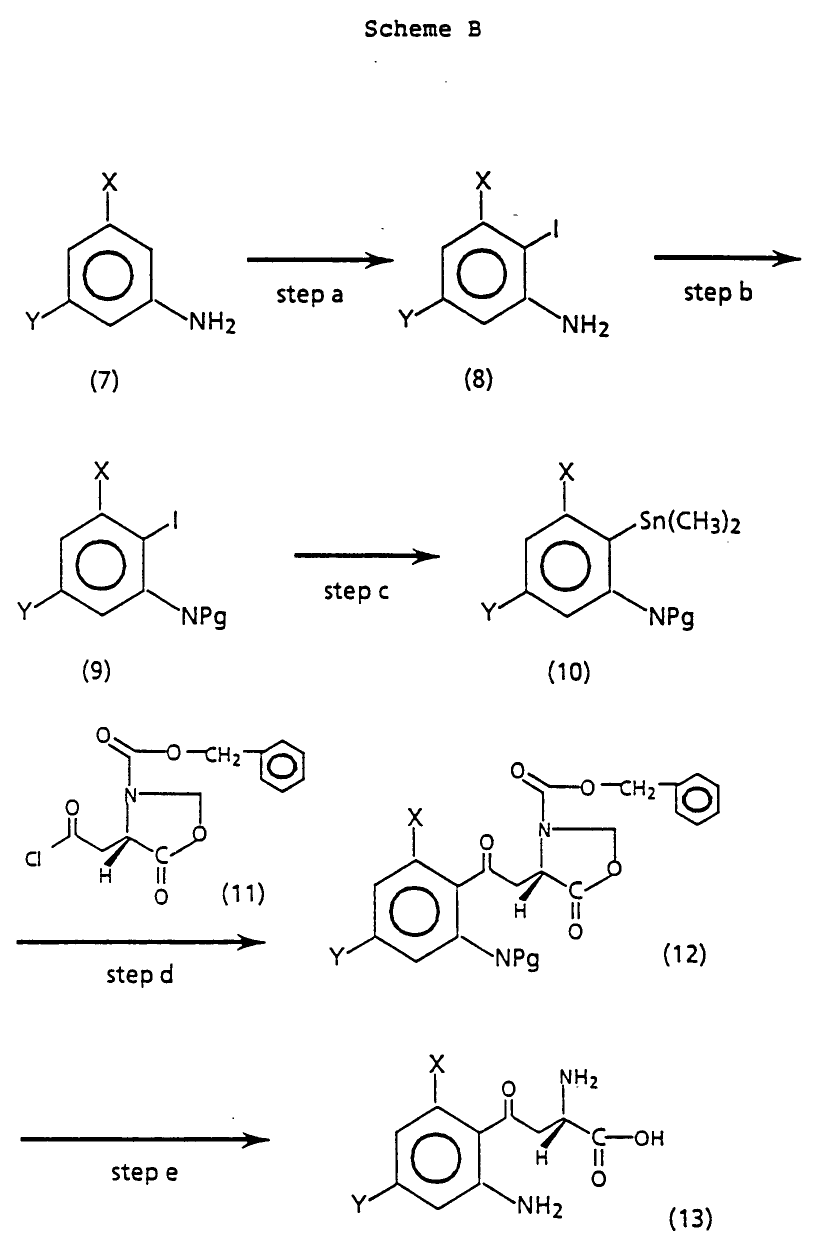
c) for those compounds Ib by carrying out the reaction depicted in Reaction Scheme C in which X and Y are as above:

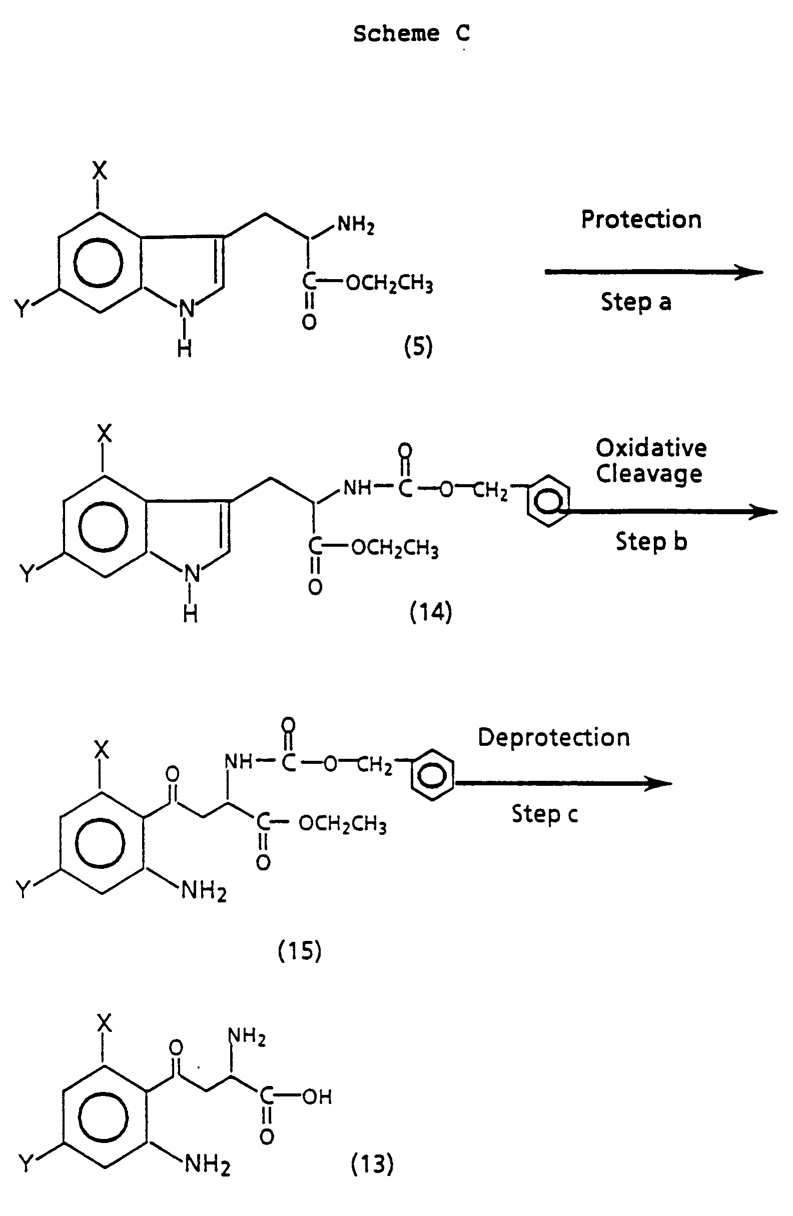

2. A process according to claim 1 in which X and Y are each represented by halogen atoms.

3. A method for the preparation of a pharmaceutical composition comprising combining a compound prepared according to claim 1 or 2 in admixture with a pharmaceutically acceptable carrier.

4. A method according to claim 3 wherein the pharmaceutical composition prepared is useful in antagonizing the effects of excitatory amino acids upon the NMDA receptor complex.

5. A method according to claim 3 wherein the pharmaceutical composition prepared is useful in the treatment of epilepsy.

6. A method according to claim 3 wherein the pharmaceutical composition prepared is useful in the treatment of neurodegenerative diseases.

7. A method according to claim 3 wherein the pharmaceutical composition prepared is useful in preventing ischemic/hypoxic/hypoglycemic damage to cerebral tissue.

8. A method according to claim 3 wherein the pharmaceutical composition prepared is useful in the treatment of anxiety.

9. A method according to claim 3 wherein the pharmaceutical composition prepared is useful in producing an analgesic effect.

10. A method according to claim 3 wherein the pharmaceutical composition prepared is useful in the treatment of migraine.

11. A method for the preparation of a pharmaceutical composition according to any one of claims 3 to 10 which additionally comprises combining probenecid with the remaining ingredients.

12. A method for the preparation of a pharmaceutical composition according to any one of claims 3 to 10 which additionally comprises the preparation of probenecid as a separate dosage form for concurrent administration.

## Claims (Claims for the following Contracting State(s): GR)

1. Compounds of the formulae: 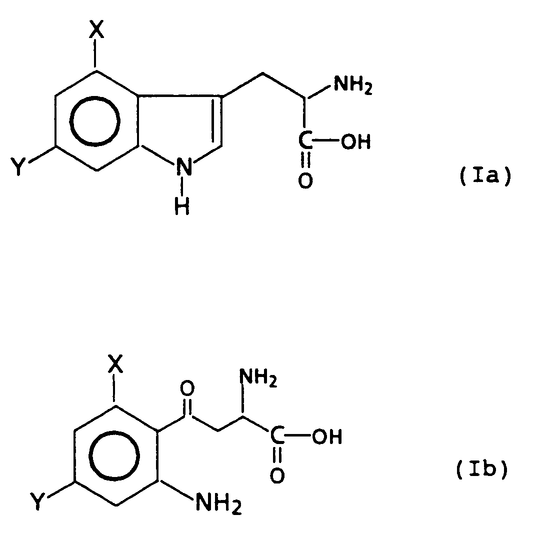 in which X and Y are each independently represented by a substituent selected from the group consisting of Cl, Br, F, CH₃, and CH₂CH₃ or a a pharmaceutically acceptable salt thereof.

2. A compound according to claim 1 in which X and Y are each represented by halogen atoms.

3. A method for the preparation of a pharmaceutical composition comprising combining a compound according to claim 1 or 2 with a pharmaceutically acceptable carrier.

4. A method according to claim 3 wherein the pharmaceutical composition prepared is useful in antagonizing the effects of excitatory amino acids upon the NMDA receptor complex.

5. A method according to claim 3 wherein the pharmaceutical composition prepared is useful in the treatment of epilepsy.

6. A method according to claim 3 wherein the pharmaceutical composition prepared is useful in the treatment of neurodegenerative diseases.

7. A method according to claim 3 wherein the pharmaceutical composition prepared is useful in preventing ischemic/hypoxic/hypoglycemic damage to cerebral tissue.

8. A method according to claim 3 wherein the pharmaceutical composition prepared is useful in the treatment of anxiety.

9. A method according to claim 3 wherein the pharmaceutical composition prepared is useful in producing an analgesic effect.

10. A method according to claim 3 wherein the pharmaceutical composition prepared is useful in the treatment of migraine.

11. A method for the preparation of a pharmaceutical composition according to any one of claims 3 to 10 which additionally comprises combining probenecid with the remaining ingredients.

12. A method for the preparation of a pharmaceutical composition according to any one of claims 3 to 10 which additionally comprises the preparation of probenecid as a separate dosage form for concurrent administration.

## Patentansprüche (Patentansprüche für folgende(n) Vertragsstaat(en): AT, BE, CH, DE, DK, FR, GB, IT, LI, LU, MC, NL, PT, SE)

1. Verbindungen der Formeln 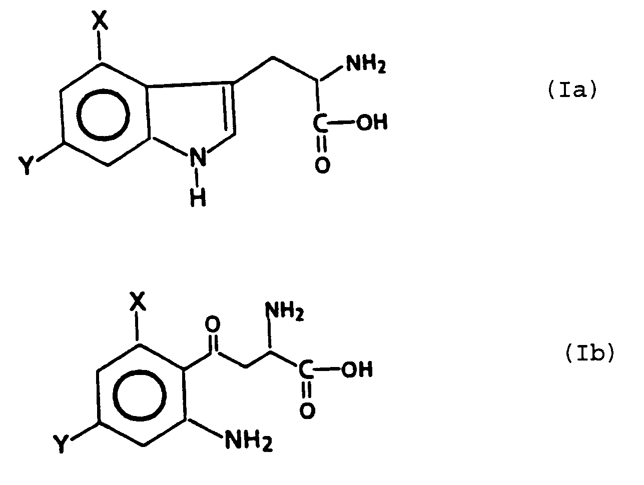 wobei X und Y jeweils unabhängig voneinander einen aus der Gruppe Cl, Br, F, CH₃ und CH₂CH₃ ausgewählten Substituenten bedeuten, sowie ein pharmazeutisch verträgliches Salz davon.

2. Verbindung nach Anspruch 1, wobei X und Y jeweils Halogenatome bedeuten.

3. Pharmazeutische Zusammensetzung, enthaltend eine Verbindung nach Anspruch 1 oder 2 im Gemisch mit einem pharmazeutisch verträglichen Träger.

4. Pharmazeutische Zusammensetzung nach Anspruch 3 zur Entfaltung eines Antagonismus gegen die Wirkung von exzitatorischen Aminosäuren auf den NMDA-Rezeptorkomplex.

5. Pharmazeutische Zusammensetzung nach Anspruch 4 zur Behandlung von Epilepsie.

6. Pharmazeutische Zusammensetzung nach Anspruch 4 zur Behandlung von neurodegenerativen Erkrankungen.

7. Pharmazeutische Zusammensetzung nach Anspruch 4 zur Verhinderung von ischämischen/hypoxischen/hypoglykämischen Schädigungen des Hirngewebes.

8. Pharmazeutische Zusammensetzung nach Anspruch 4 zur Behandlung von Angstzuständen.

9. Pharmazeutische Zusammensetzung nach Anspruch 4 zur Herbeiführung einer analgetischen Wirkung.

10. Pharmazeutische Zusammensetzung nach Anspruch 4 zur Behandlung von Migräne.

11. Pharmazeutische Zusammensetzung, enthaltend eine pharmazeutische Zusammensetzung nach einem der Ansprüche 3 bis 10 im Gemisch mit Probenecid.

12. Pharmazeutische Zusammensetzung nach einem der Ansprüche 3 bis 10, enthaltend zusätzlich Probenecid in einer separaten Dosierungsform zur gleichzeitigen Verabreichung.

## Patentansprüche (Patentansprüche für folgende(n) Vertragsstaat(en): ES)

1. Verfahren zur Herstellung von Verbindungen der Formeln 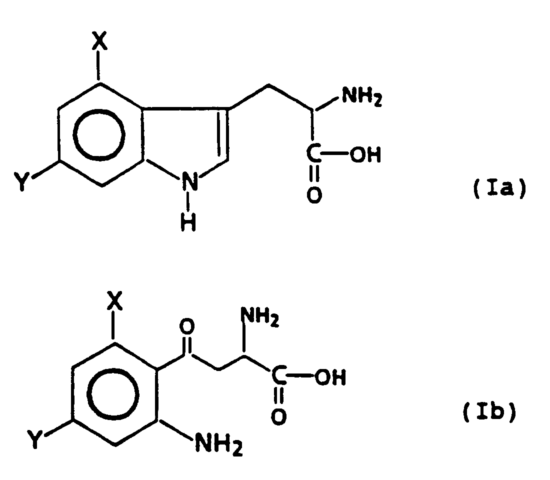 wobei X und Y jeweils unabhängig voneinander einen aus der Gruppe Cl, Br, F, CH₃ und CH₂CH₃ ausgewählten Substituenten bedeuten, sowie ein pharmazeutisch verträgliches Salz davon,
umfassend:
a) für die Verbindungen Ia die Durchführung der im Reaktionsschema A dargestellten Reaktion, wobei X und Y die vorstehenden Bedeutungen haben, 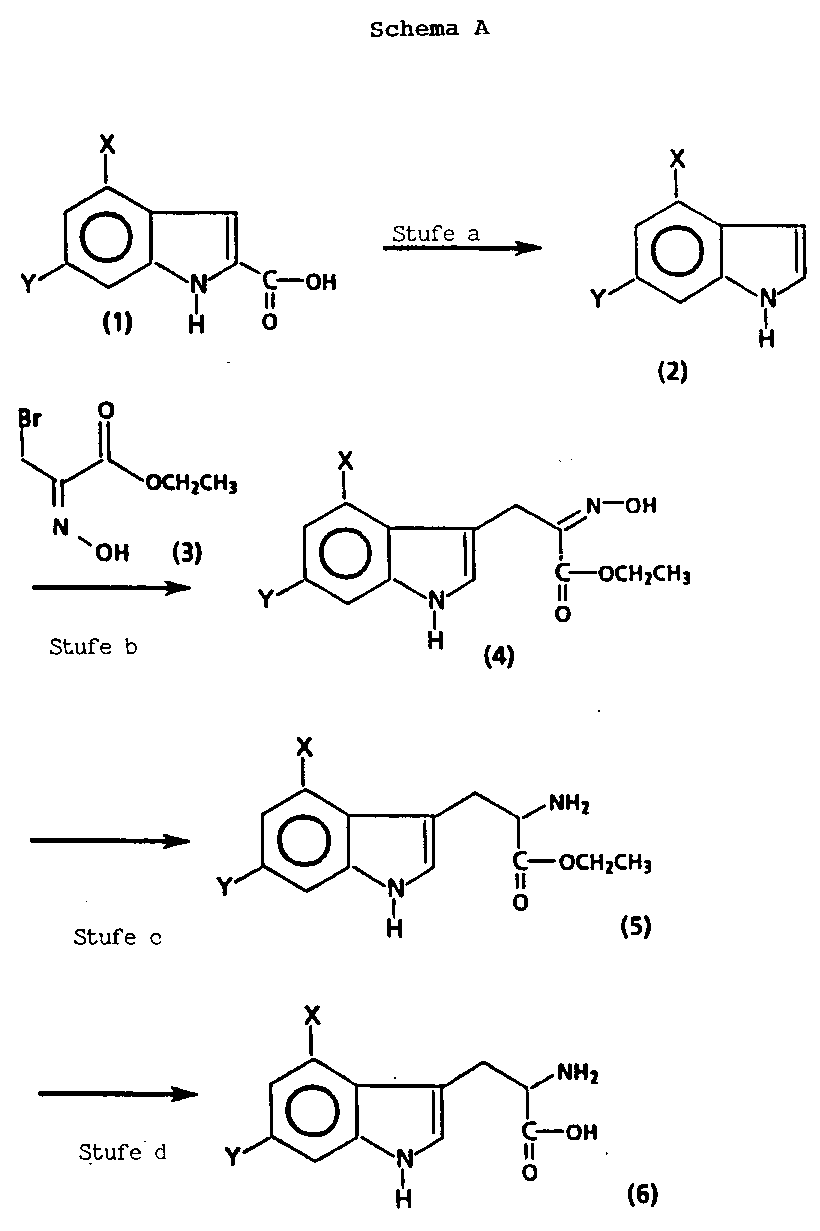
b) für die Verbindungen Ib die Durchführung der im Reaktionsschema B dargestellten Reaktion, wobei X und Y die vorstehenden Bedeutungen haben, 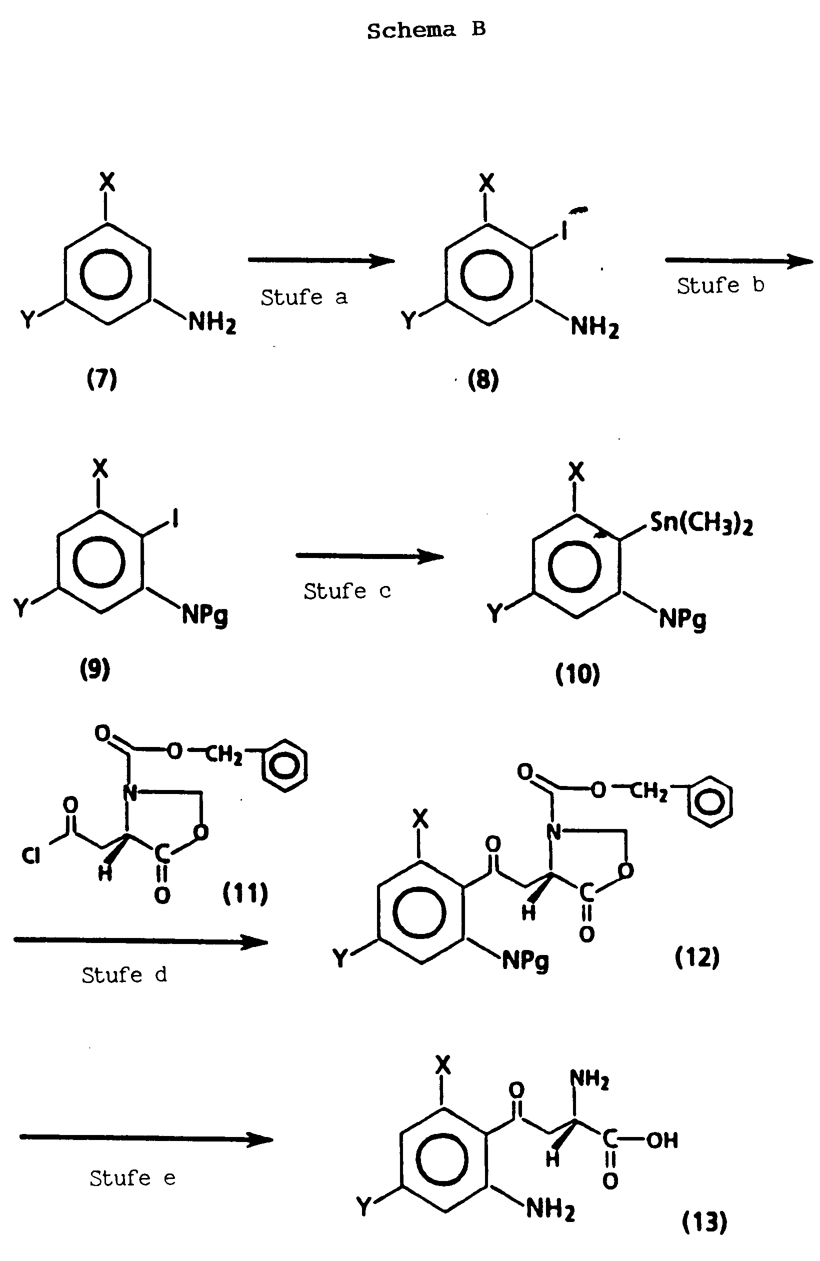
c) Für die Verbindungen Ib die Durchführung der im Reaktionsschema C dargestellten Reaktion, wobei X und Y die vorstehenden Bedeutungen haben,

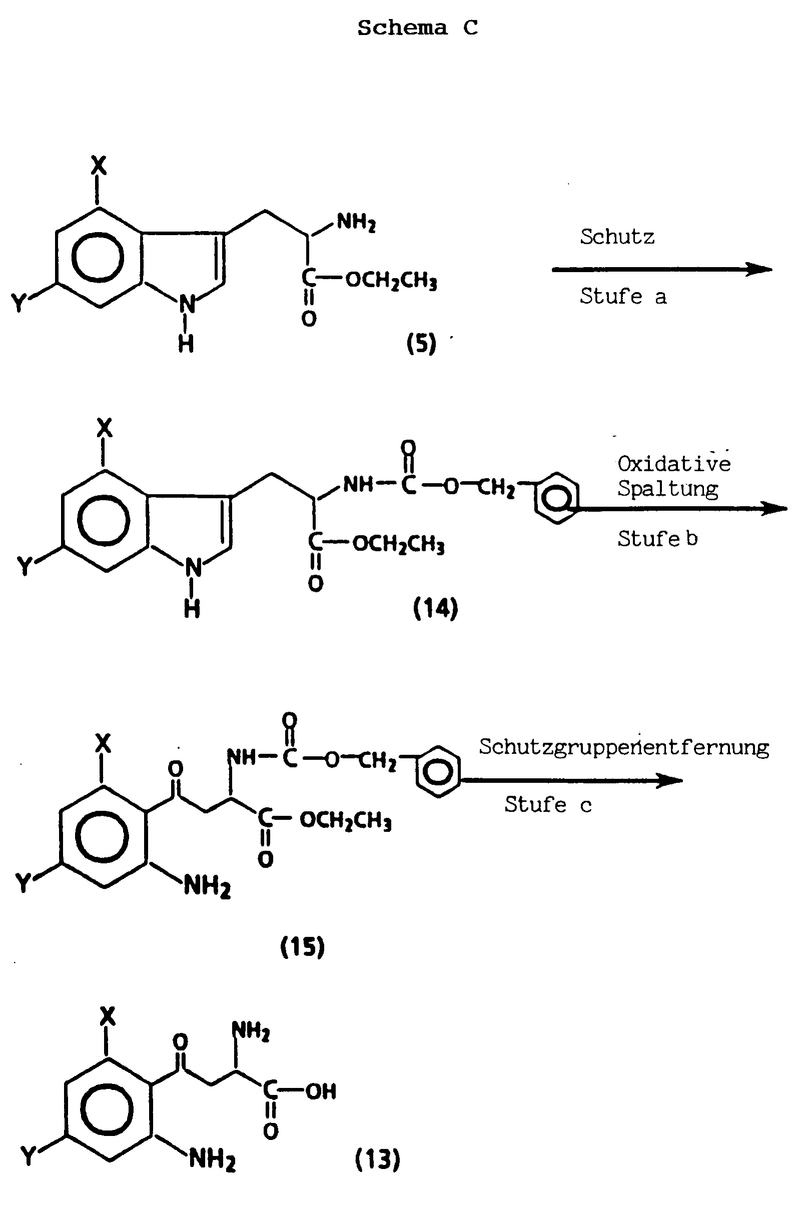

2. Verfahren nach Anspruch 1, wobei X und Y jeweils Halogenatome bedeuten.

3. Verfahren zur Herstellung einer pharmazeutischen Zusammensetzung, umfassend die Vereinigung einer gemäß Anspruch 1 oder 2 hergestellten Verbindung mit einem pharmazeutisch verträglichen Träger.

4. Verfahren nach Anspruch 3, wobei die hergestellte pharmazeutische Zusammensetzung sich zur Entfaltung eines Antagonismus gegen die Wirkung von exzitatorischen Aminosäuren auf den NMDA-Rezeptorkomplex eignet.

5. Verfahren nach Anspruch 3, wobei die hergestellte pharmazeutische Zusammensetzung sich zur Behandlung von Epilepsie eignet.

6. Verfahren nach Anspruch 3, wobei die hergestellte pharmazeutische Zusammensetzung sich zur Behandlung von neurodegenerativen Erkrankungen eignet.

7. Verfahren nach Anspruch 3, wobei die hergestellte pharmazeutische Zusammensetzung sich zur Verhinderung von ischämischen/hypoxischen/hypoglykämischen Schädigungen des Hirngewebes eignet.

8. Verfahren nach Anspruch 3, wobei die hergestellte pharmazeutische Zusammensetzung sich zur Behandlung von Angstzuständen eignet.

9. Verfahren nach Anspruch 3, wobei die hergestellte pharmazeutische Zusammensetzung sich zur Herbeiführung einer analgetischen Wirkung eignet.

10. Verfahren nach Anspruch 3, wobei die hergestellte pharmazeutische Zusammensetzung sich zur Behandlung von Migräne eignet.

11. Verfahren zur Herstellung einer pharmazeutischen Zusammensetzung nach einem der Ansprüche 3 bis 10, das zusätzlich die Vereinigung von Probenecid mit den restlichen Bestandteilen umfaßt.

12. Verfahren zur Herstellung einer pharmazeutischen Zusammensetzung nach einem der Ansprüche 3 bis 10, das zusätzlich die Herstellung eines Probenecidpräparats als separate Dosierungsform für die gleichzeitige Verabreichung umfaßt.

## Patentansprüche (Patentansprüche für folgende(n) Vertragsstaat(en): GR)

1. Verbindungen der Formeln 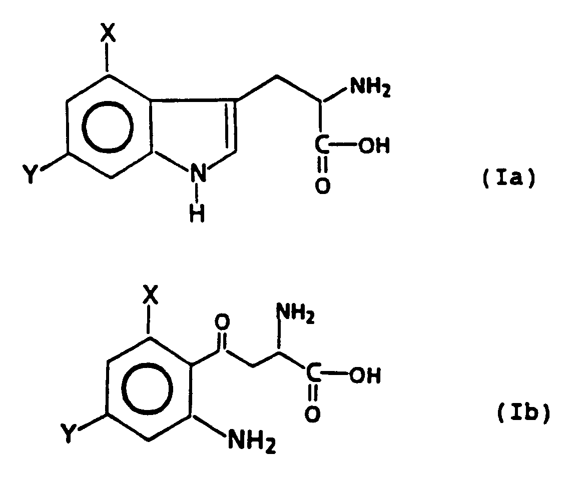 wobei X und Y jeweils unabhängig voneinander einen aus der Gruppe Cl, Br, F, CH₃ und CH₂CH₃ ausgewählten Substituenten bedeuten, sowie ein pharmazeutisch verträgliches Salz davon.

2. Verbindung nach Anspruch 1, wobei X und Y jeweils Halogenatome bedeuten.

3. Verfahren zur Herstellung einer pharmazeutischen Zusammensetzung, umfassend die Vereinigung einer Verbindung nach Anspruch 1 oder 2 mit einem pharmazeutisch verträglichen Träger.

4. Verfahren nach Anspruch 3, wobei die hergestellte pharmazeutische Zusammensetzung sich zur Entfaltung eines Antagonismus gegen die Wirkung von exzitatorischen Aminosäuren auf den NMDA-Rezeptorkomplex eignet.

5. Verfahren nach Anspruch 3, wobei die hergestellte pharmazeutische Zusammensetzung sich zur Behandlung von Epilepsie eignet.

6. Verfahren nach Anspruch 3, wobei die hergestellte pharmazeutische Zusammensetzung sich zur Behandlung von neurodegenerativen Erkrankungen eignet.

7. Verfahren nach Anspruch 3, wobei die hergestellte pharmazeutische Zusammensetzung sich zur Verhinderung von ischämischen/hypoxischen/hypoglykämischen Schädigungen des Hirngewebes eignet.

8. Verfahren nach Anspruch 3, wobei die hergestellte pharmazeutische Zusammensetzung sich zur Behandlung von Angstzuständen eignet.

9. Verfahren nach Anspruch 3, wobei die hergestellte pharmazeutische Zusammensetzung sich zur Herbeiführung einer analgetischen Wirkung eignet.

10. Verfahren nach Anspruch 3, wobei die hergestellte pharmazeutische Zusammensetzung sich zur Behandlung von Migräne eignet.

11. Verfahren zur Herstellung einer pharmazeutischen Zusammensetzung nach einem der Ansprüche 3 bis 10, das zusätzlich die Vereinigung von Probenecid mit den restlichen Bestandteilen umfaßt.

12. Verfahren zur Herstellung einer pharmazeutischen Zusammensetzung nach einem der Ansprüche 3 bis 10, das zusätzlich die Herstellung eines Probenecidpräparats als separate Dosierungsform für die gleichzeitige Verabreichung umfaßt.

## Revendications (Revendications pour l'(les) Etat(s) contractant(s) suivant(s): AT, BE, CH, DE, DK, FR, GB, IT, LI, LU, MC, NL, PT, SE)

1. Composés selon les formules: 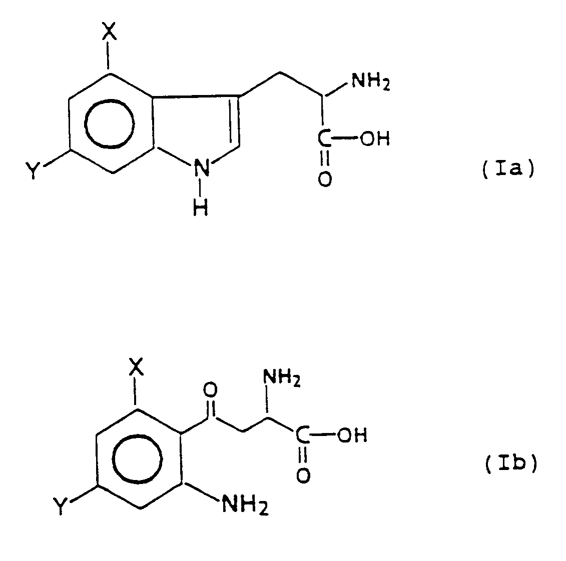 dans lesquelles X et Y sont représentés chacun indépendamment par un substituant choisi dans le groupe consistant en Cl, Br, F, CH₃ et CH₂CH₃ ou sel pharmaceutiquement acceptable de ceux-ci.

2. Composé selon la revendication 1, dans lequel X et Y sont représentés chacun par des atomes d'halogène.

3. Composition pharmaceutique comprenant un composé selon la revendication 1 ou 2 en mélange avec un véhicule pharmaceutiquement acceptable.

4. Composition pharmaceutique selon la revendication 3, utile pour antagoniser les effets d'acides aminés excitateurs sur le complexe des récepteurs du NMDA.

5. Composition pharmaceutique selon la revendication 4, utile dans le traitement de l'épilepsie.

6. Composition pharmaceutique selon la revendication 4, utile dans le traitement des maladies neurodégénératives.

7. Composition pharmaceutique selon la revendication 4, utile dans la prévention des lésions ischémiques/hypoxiques/hypoglycémiques du tissu cérébral.

8. Composition pharmaceutique selon la revendication 4, utile dans le traitement de l'anxiété.

9. Composition pharmaceutique selon la revendication 4, utile dans la production d'un effet analgésique.

10. Composition pharmaceutique selon la revendication 4, utile dans le traitement de la migraine.

11. Composition pharmaceutique comprenant une composition pharmaceutique selon l'une quelconque des revendications 3 à 10 en mélange avec du probénécide.

12. Composition pharmaceutique selon l'une quelconque des revendications 3 à 10, comprenant en outre du probénécide dans une forme galénique séparée pour une administration concordante.

## Revendications (Revendications pour l'(les) Etat(s) contractant(s) suivant(s): ES)

1. Procédé de préparation de composés selon les formules: 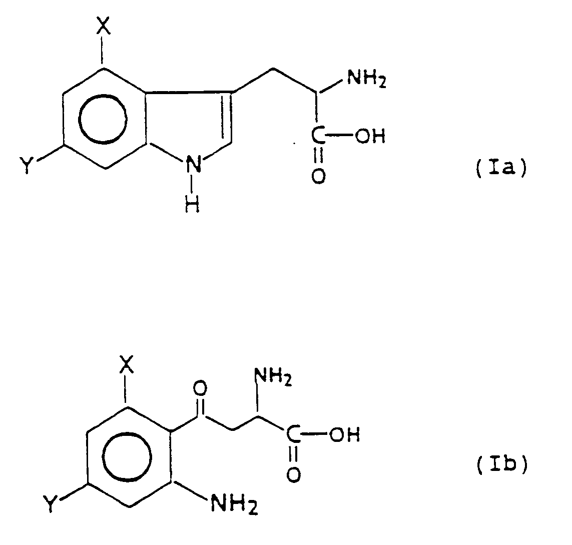 dans lesquelles X et Y sont représentés chacun indépendamment par un substituant choisi dans le groupe consistant en Cl, Br, F, CH₃ et CH₂CH₃ ou d'un sel pharmaceutiquement acceptable de ceux-ci, comprenant:
a) pour les composés Ia, la mise en oeuvre de la réaction représentée dans le schéma réactionnel A dans lequel X et Y sont tels que ci-dessus: 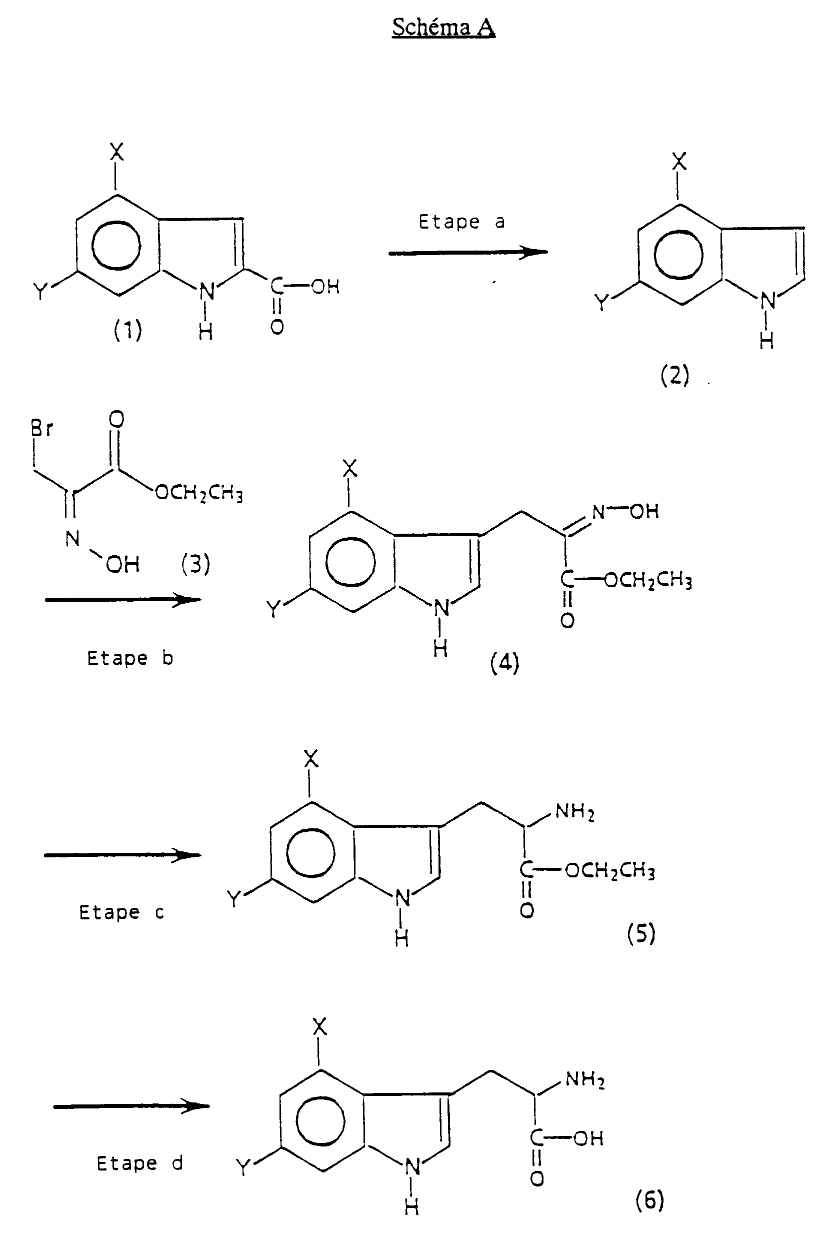
b) pour les composés Ib, la mise en oeuvre de la réaction représentée dans le schéma réactionnel B dans lequel X et Y sont tels que ci-dessus: 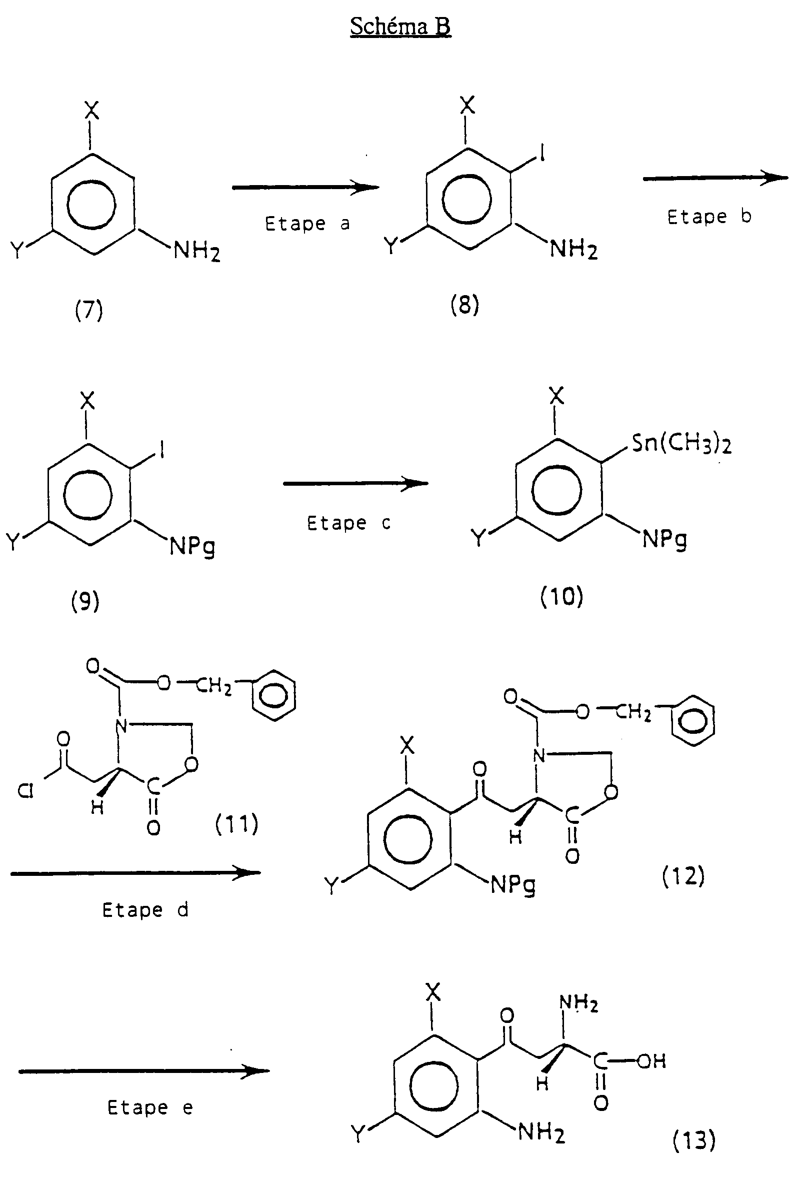
c) pour les composés Ib, la mise en oeuvre de la réaction représentée dans le schéma réactionnel C dans lequel X et Y sont tels que ci-dessus:

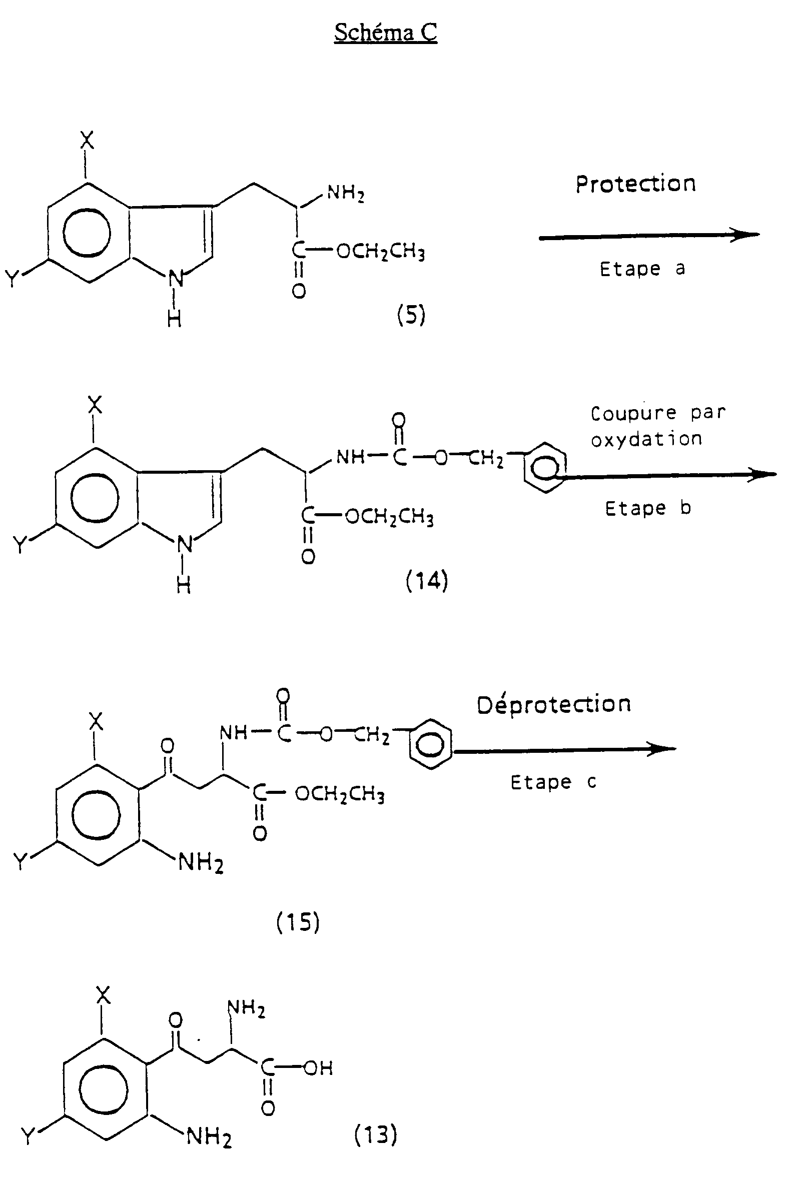

2. Procédé selon la revendication 1, dans lequel X et Y sont représentés chacun par des atomes d'halogène.

3. Procédé de préparation d'une composition pharmaceutique, comprenant la combinaison d'un composé préparé selon la revendication 1 ou 2 en mélange avec un véhicule pharmaceutiquement acceptable.

4. Procédé selon la revendication 3, dans lequel la composition pharmaceutique préparée est utile pour antagoniser les effets d'acides aminés excitateurs sur le complexe des récepteurs du NMDA.

5. Procédé selon la revendication 3, dans lequel la composition pharmaceutique préparée est utile dans le traitement de l'épilepsie.

6. Procédé selon la revendication 3, dans lequel la composition pharmaceutique préparée est utile dans le traitement des maladies neurodégénératives.

7. Procédé selon la revendication 3, dans lequel la composition pharmaceutique préparée est utile dans la prévention des lésions ischémiques/hypoxiques/hypoglycémiques du tissu cérébral.

8. Procédé selon la revendication 3, dans lequel la composition pharmaceutique préparée est utile dans le traitement de l'anxiété.

9. Procédé selon la revendication 3, dans lequel la composition pharmaceutique préparée est utile dans la production d'un effet analgésique.

10. Procédé selon la revendication 3, dans lequel la composition pharmaceutique préparée est utile dans le traitement de la migraine.

11. Procédé de préparation d'une composition pharmaceutique selon l'une quelconque des revendications 3 à 10 qui comprend en outre la combinaison de probénécide avec les ingrédients restants.

12. Procédé de préparation d'une composition pharmaceutique selon l'une quelconque des revendications 3 à 10, qui comprend en outre la préparation de probénécide en tant que forme galénique séparée pour une administration concordante.

## Revendications (Revendications pour l'(les) Etat(s) contractant(s) suivant(s): GR)

1. Composés selon les formules : 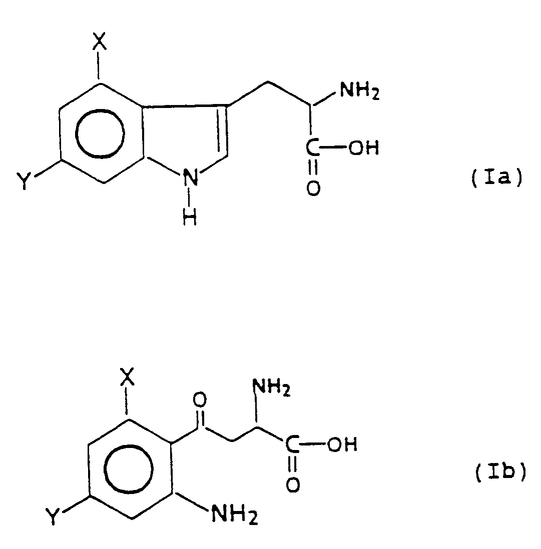 dans lesquelles X et Y sont représentés chacun indépendamment par un substituant choisi dans le groupe consistant en Cl, Br, F, CH₃ et CH₂CH₃ ou sel pharmaceutiquement acceptable de ceux-ci.

2. Composé selon la revendication 1, dans lequel X et Y sont représentés chacun par des atomes d'halogène.

3. Procédé de préparation d'une composition pharmaceutique comprenant la combinaison d'un composé selon la revendication 1 ou 2 avec un véhicule pharmaceutiquement acceptable.

4. Procédé selon la revendication 3, dans lequel la composition pharmaceutique préparée est utile pour antagoniser les effets d'acides aminés excitateurs sur le complexe des récepteurs du NMDA.

5. Procédé selon la revendication 3, dans lequel la composition pharmaceutique préparée est utile dans le traitement de l'épilepsie.

6. Procédé selon la revendication 3, dans lequel la composition pharmaceutique préparée est utile dans le traitement des maladies neurodégénératives.

7. Procédé selon la revendication 3, dans lequel la composition pharmaceutique préparée est utile dans la prévention des lésions ischémiques/hypoxiques/hypoglycémiques du tissu cérébral.

8. Procédé selon la revendication 3, dans lequel la composition pharmaceutique préparée est utile dans le traitement de l'anxiété.

9. Procédé selon la revendication 3, dans lequel la composition pharmaceutique préparée est utile dans la production d'un effet analgésique.

10. Procédé selon la revendication 3, dans lequel la composition pharmaceutique préparée est utile dans le traitement de la migraine.

11. Procédé de préparation d'une composition pharmaceutique selon l'une quelconque des revendications 3 à 10 qui comprend en outre la combinaison de probénécide avec les ingrédients restants.

12. Procédé de préparation d'une composition pharmaceutique selon l'une quelconque des revendications 3 à 10, qui comprend en outre la préparation de probénécide en tant que forme galénique séparée pour une administration concordante.
